(19) Europäisches Patentamt / European Patent Office / Office européen des brevets

(11) **EP 4 013 466 B1**

(12) **EUROPEAN PATENT SPECIFICATION**

(45) Date of publication and mention
of the grant of the patent:
**16.10.2024 Bulletin 2024/42**

(21) Application number: **20750291.5**

(22) Date of filing: **07.08.2020**

(51) International Patent Classification (IPC):
**A61L 27/36** *(2006.01)*        **A61L 27/38** *(2006.01)*
**C12N 5/077** *(2010.01)*

(52) Cooperative Patent Classification (CPC):
**A61L 27/3612; A61L 27/3654; A61L 27/3683;
A61L 27/3817; A61L 27/3852; A61L 27/3895;
C12N 5/0655;** A61L 2430/06; C12N 2501/115;
C12N 2501/15; C12N 2501/415; C12N 2506/13;
C12N 2513/00

(86) International application number:
**PCT/EP2020/072237**

(87) International publication number:
**WO 2021/028335 (18.02.2021 Gazette 2021/07)**

(54) **METHOD FOR IN VITRO PRODUCTION OF HYALINE CARTILAGE TISSUE**

VERFAHREN ZUR IN-VITRO-HERSTELLUNG VON HYALINKNORPELGEWEBE

MÉTHODE DE PRODUCTION IN VITRO DE TISSU CARTILAGINEUX HYALIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.08.2019 EP 19191756**

(43) Date of publication of application:
**22.06.2022 Bulletin 2022/25**

(73) Proprietor: **Vanarix SA
1005 Lausanne (CH)**

(72) Inventor: **TIENG, Vannary
1205 GENEVE (CH)**

(74) Representative: **Icosa
83 avenue Denfert-Rochereau
75014 Paris (FR)**

(56) References cited:
**US-A1- 2017 274 018**

• **TAY A G ET AL: "Cell yield, proliferation, and postexpansion differentiation capacity of human ear, nasal, and rib chondrocytes", TISSUE ENGINEERING, LARCHMONT, NY, US, vol. 10, no. 5-6, 1 May 2004 (2004-05-01), pages 762 - 770, XP002557029, ISSN: 1076-3279, [retrieved on 20040825], DOI: 10.1089/1076327041348572**
• **MARTIN I ET AL: "MAMMALIAN CHONDROCYTES EXPANDED IN THE PRESENCE OF FIBROBLAST GROWTH FACTOR 2 MAINTAIN THE ABILITY TO DIFFERENTIATE AND REGENERATE THREE-DIMENSIONAL CARTILAGINOUS TISSUE", EXPERIMENTAL CELL RESEARCH, ELSEVIER, AMSTERDAM, NL, vol. 253, no. 2, 15 December 1999 (1999-12-15), pages 681 - 688, XP000901329, ISSN: 0014-4827, DOI: 10.1006/EXCR.1999.4708**

**Description**

**FIELD OF THE INVENTION**

The present invention relates to a novel method for *in vitro* production of hyaline cartilage tissue ("Cartibeads"), and to therapeutic uses and screening methods using the cartilage tissue thus produced.

**BACKGROUND OF THE INVENTION**

Hyaline cartilage is composed of specialized cells called chondrocytes, surrounded by an extracellular matrix. This matrix is synthesized and secreted by the chondrocytes and is composed mainly of type II collagen fibers, glycosaminoglycan (GAG), and 60-80% of water. Hyaline cartilage has four layers: superficial, intermediate, deep and calcified layers on top of the subchondral bone. The biomechanical properties of the joint cartilage are largely dependent on the composition and integrity of the extracellular matrix.

Cartilage has a very limited capacity for self-repair and upon injury often evolves toward osteoarthritis (OA). Aging and repetitive trauma (e.g., occurring during intensive sport practice) are major risk factors for the degeneration of knee cartilage. OA affects a large number of people and occurs most often in older people (prevalence >10% in people over 60), while younger people are generally affected by OA following joint injuries.

Surgery treatments involve joint replacement by prosthesis. However, the lifetime for prosthesis is limited to 15-20 years. Further, pain relief is not completely attenuated for the majority of patients: 20 to 30% of patients with knee prosthesis still continue to feel discomfort or pain. Treatments are mostly palliative and target pain relief. Some biologic agents like mesenchymal stem cells, hyaluronic acid or platelet rich plasma injection can delay joint deterioration, but they do not promote tissue regeneration.

For the moment, strategies using external and synthetic scaffolds to fill the defect are not satisfactory and incompletely mimic the biomechanical properties of articular cartilage. Classical treatments include micro-fracture surgery to stimulate migration of stem cells to the lesioned area, or direct implantation of chondrocytes.

The microfracture procedure consists on making tiny holes into the subchondral bone with the objective to stimulate the migration of mesenchymal stem cells from the bone medulla inside a coagulum to form new chondrocytes and replace damaged tissue.

In autologous chondrocytes implantation (ACI), chondrocytes were extracted from cartilage, cultures in a limited number of passages and transplanted into the lesion. To improve the method, matrix such as type I/III pig collagen or hyaluronic acid for culturing the chondrocytes can be used (Kon E, et al., 2009, The American journal of sports medicine 37(1):33-41; Hettrich CM, Crawford D, & Rodeo SA, 2008, 16(4):230-235). These matrices are either applied directly on the lesion after microfracture procedure or used *in vitro* to culture chondrocytes prior to re-implantation (Ekkers JE, et al. 2013, Osteoarthritis Research Society 21(7):950-956).

Cell-based therapies using autologous chondrocyte transplantation (ACI) are indicated for cartilage lesions greater than two square centimeters (Armoiry X. et al. 2019, Pharmacoeconomics 37, 879-886). Chondrocytes represent a logical cell source for cartilage regeneration. Indeed, only this type of cartilage cells is involved in the maintenance of the hyaline cartilage matrix.

A major challenge is that chondrocytes tend to dedifferentiate into fibroblast-like cells in culture, resulting in a rapid loss of function usually occurring at the second or third cell passage (Munirah, S. et al. 2010. Tissue & cell 42, 282-292) in 2-dimension cell culture systems. Dedifferentiated chondrocytes are characterized by the loss of glycosaminoglycan (GAG) and type II collagen production, the main constituents of hyaline cartilage (Wu, L. et al. 2014. Tissue Eng Part C Methods 20, 160-168; Benya, P. D et al. 1978. Cell. 15, 1313-1321) which are replaced by type I collagen, present in the fibrocartilage. Indeed, during amplification step, chondrocytes tend to dedifferentiate by losing their original phenotype and become fibroblast-like cells (elongated cells) with stem cell-like features in gene expression (expression of cell surface markers such as CD73, CD90, CD105 used to characterize mesenchymal stem cells (MSC)). For instance, chondrocytes extracted from patient's articular cartilage lose rapidly their chondrogenic capacity to synthesize specific hyaline matrix cartilage after few passages in *in vitro* culture. Fibrocartilage is biomechanically different from hyaline cartilage and is not considered as an effective long-lasting treatment because it leads to a dysfunctional repair. In contrast, chondrocyte hyaline properties are known to be preserved in three-dimensional culture systems (Benya, P. D. & Shaffer, J. D. 1982. Cell 30, 215-224), mimicking *in vivo* environment. Tay A G et al. discloses human ear, nasal and rib chondrocytes were compared with respect to their suitability to generate autologous cartilage grafts. Cells were expanded for two passages in medium containing 10% fetal bovine serum without (control) or with transforming growth factor β1 (TGF-β1), fibroblast growth factor 2 (FGF-2), and PDGF-bb (TFP). Expanded cells were cultured as three-dimensional pellets in chondrogenic serum-free medium containing insulin, dexamethasone, and TGF-β1. Chondrocytes from all three sources were successfully isolated, increased their proliferation rate in response to TFP, and dedifferentiated during passaging (Tissue Engineering, Larchmont, NY, US, vol. 10. no. 5-6., pages 762-770).

The loss of chondrogenic capacity in culture is particularly true in old patients. Thus, another challenge for chondrocyte based-cell therapy is the difficulty to expand and differentiate cells from elderly patients, limiting their use in this population. Consequently, most clinical trials limit the inclusion of patients to age 55.

The idea of using chondrocytes to produce cartilage makes sense if cell amplification could be done along with original phenotype maintenance. Thus, it remains a need to develop a standardized method for cartilage production which results in high quality hyaline-like cartilage tissue (characterized by high level of GAG detection), which mimics intrinsic properties for patients of all ages

## SUMMARY OF THE INVENTION

The main achievement of the present study is a method that can reverse the loss of chondrocyte phenotype (dedifferentiation of chondrocytes) during expansion. This solves a key problem encountered in cell therapy using chondrocytes as the starting material. The present data demonstrates that a novel 3-step method can produce a high-quality cartilage with hyaline characteristics, regardless of the patient age and the joint's arthritic status.

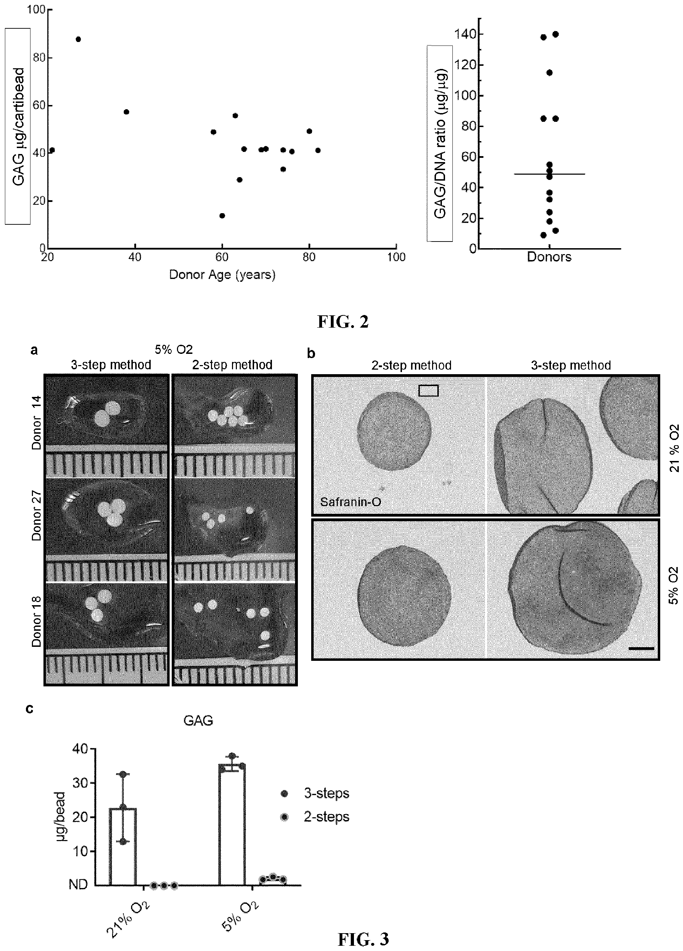

The Cartibead method allows cell expansion from a very small sample of cartilage harvest (~30 mg in our preclinical minipig study), as opposed to 260 mg on average in conventional human chondrocyte-based cell therapy (Brittberg, M. 2018. Injury 39 Suppl 1, S40-49), which reduces donor site morbidity. The present method showed a ratio of GAG/Cartibead that is 20 times more than previously reported cartilage microtissues, suggesting that these cartilage microtissues are less hyaline and contained more fibrocartilage (Bartz, C. et al. 2016. J Transl Med 14, 317). Consistent with these results, the inventor obtained on average a ratio of GAG/DNA at least three times higher than other published method (**Figure 2B**) (Mumme, M. et al. 2016. Lancet 388, 1985-1994; Dang, P. N et al. 2014. Tissue engineering. Part A 20, 3163-3175).

Redifferentiation was due to removal of FGF-2. However, its removal in 3D culture was not sufficient to induce hyaline matrix synthesis in the 2-step method. Redifferentiation of chondrocytes most likely requires cell adhesion to a matrix coated-flask and induction of specific cell signaling pathways in 2D culture.

The inventor has developed a novel method to amplify chondrocytes in culture and make them recover their chondrogenic capacity independently of patient age from a small piece of cartilage (~ 30 mg). In a first step, cells are amplified in two-dimensional culture on extracellular matrix with the support of FGF-2, then pre-redifferentiated towards the original phenotype in a second step, by removing FGF-2 used for cell amplification. In a final step, complete redifferentiation is achieved in three-dimensional (3D) culture with spontaneous cell aggregation to form microtissues. By removing FGF-2 growth factor in the second step specifically in 2D culture, the inventor has surprisingly shown that chondrocytes begin to recover their original function by synthesizing aggrecan, a protein associated with GAG, and by maintaining FGF-2 removal in the third step in 3D culture, collagen II is synthetized in combination of GAG, hence producing the hyaline matrix specific of articular cartilage. The inventor further showed that the removal of FGF-2 induces the inactivation of Wnt signaling pathway, preferably Wnt7B signaling after amplification step and thus allows the production of homogeneous hyaline cartilage that is highly representative of *in vivo* cartilage. The inventor showed that Wnt7B is activated in dedifferentiation medium (ME) and decreases at least 10-fold in pre-redifferentiation medium (MR) and at least 40-fold in maturation medium (MI). Transplantation of high-quality hyaline microtissues, with characteristics as close as possible to those of native cartilage, is key to envisage long-term success in the treatment of cartilage lesions (Negoro, T et al. 2018. npj Regenerative Medicine 3, 17; Madeira, C et al. 2015. Trends in biotechnology 33, 35-42).

The engineered cartilage ("Cartibeads") therefore has a high potential for full integration into the cartilage lesion following transplantation.

Thus, the present invention relates to a method for *in vitro* production of hyaline cartilage tissue comprising a first step of culturing chondrocytes, preferably chondrocytes isolated from a subject, more preferably from cartilage tissue of a human or horse subject, on an adherent culture system in a dedifferentiation culture medium that activates Wnt signaling pathway to obtain fibroblastic-like cells, preferably during 10 to 15 days; a second step of culturing said fibroblastic-like chondrocytes on an adherent culture system in a redifferentiation culture medium that inactivates Wnt signaling pathway to obtain chondrocytes with full capacity to resynthesize hyaline matrix, preferably during 4 to 8 days; and a third step of culturing said chondrocytes obtained in the second step in a three-dimensional culture system in an induction/maturation culture medium that maintains the inactivation of Wnt signaling pathway, preferably during 10 to 15 days.

In a preferred embodiment, said dedifferentiation culture medium comprises FGF-2, and said redifferentiation culture medium and induction/maturation culture medium are FGF-2 free media. Said dedifferentiation culture medium can comprise at least one growth factor selected from the group consisting of: PDGF-BB, TGF-β, and EGF. Said redifferentiation culture medium and said induction/maturation culture medium can comprise TGF-β, preferably TGF-β3, more preferably TGF-β3 and FGF-7. In another embodiment said redifferentiation culture medium can comprise platelet lysate. In particular, all the culture media as described above can comprise serum. Said induction/maturation culture medium can comprise at least one component selected from the group consisting of: insulin, IGF-1, BMP-2, selenium, transferrin and ethanolamine.

In a particular embodiment, said chondrocytes are cultured in the third step in hypoxia atmosphere comprising less than 10% $O_2$ (v/v). In an embodiment, said chondrocytes are cultured in step i) during 10 to 15 days, in step ii) during 4 to 8 days, and/or in step iii) during 10 to 15 days. In an embodiment, said chondrocytes of step i) are chondrocytes isolated from a subject, preferably from cartilage tissue of a human or horse subject.

In another aspect, the present invention relates to an engineered cartilage tissue in a form of spheroid (Cartibeads) presenting a glycosaminoglycan (GAG) content per spheroid between 10 and 100 µg/spheroid obtainable by the method as described above. The present invention also relates to a pharmaceutical composition comprising said engineered cartilage tissue and one or more pharmaceutically acceptable excipients.

In another aspect, the present invention relates to the engineered cartilage tissue as described above or to the pharmaceutical composition as described above for use in the treatment of cartilage defects and cartilage degenerative disease in a subject in need thereof. In an embodiment, said engineered cartilage tissue or pharmaceutical composition is for use in autologous transplantation in a subject in need thereof. In another embodiment, said engineered cartilage tissue or pharmaceutical composition is for use in allogenic transplantation in a subject in need thereof.

Finally, the present invention also relates to a method of screening molecules inhibiting the cartilage degenerative process comprising contacting the engineered cartilage tissue as described above with one or more candidate molecules and selecting molecules inhibiting the cartilage degenerative process.

## BRIEF DESCRIPTION OF THE FIGURES

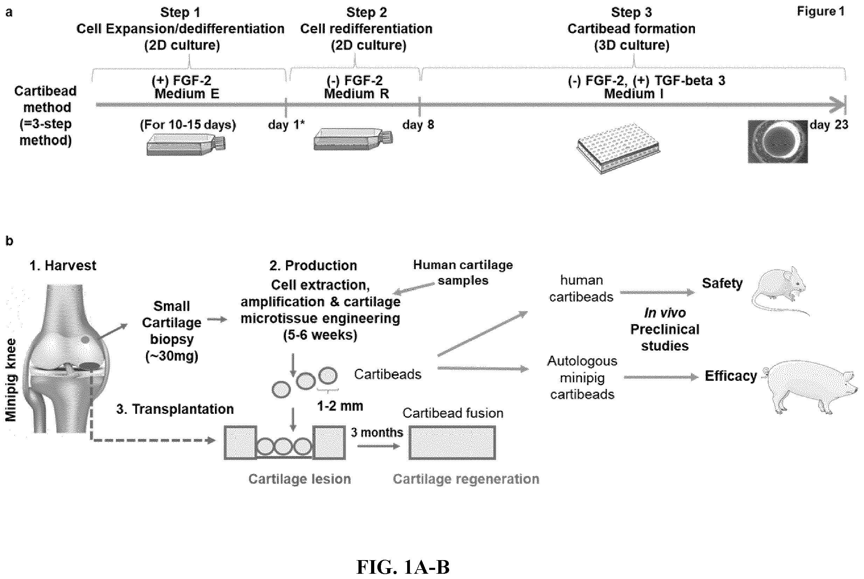

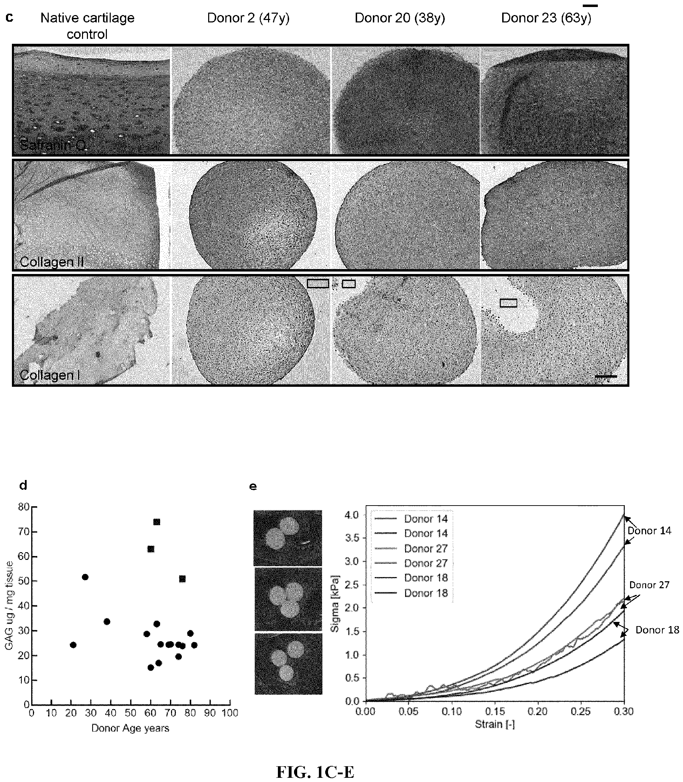

**Figure 1. Characterization of Cartibeads engineered in a 3-step method. a,** Scheme of the 3-step method for the generation of Cartibeads derived from dedifferentiated chondrocytes: step 1 (expansion), step 2 (redifferentiation) were performed in 2D and in atmospheric conditions of oxygen (21%), step 3 (Cartibead formation) was performed in 3D culture and in hypoxic conditions of oxygen (5%). **b,** figure of the preclinical studies: use of human Cartibeads to evaluate its safety in SCID mice and efficacy in minipig. **c,** Histological analysis of Cartibeads from fixed samples. Representative images of Safranin-O staining of GAG (top panel) and strong immunodetection of type II collagen (DAB staining, middle) characterizing hyaline cartilage, and weak type I collagen detection (low panel) of Cartibeads obtained from 3 independent donors. Scale bar 200 µm. **d,** Biochemical quantification of glycosaminoglycan (GAG) content in Cartibeads expressed in µg/mg tissue, determined with dimethylmethylene blue assay (DMMB), from 15 donors (black dots) and from 3 native cartilage controls (square) **e,** Biomechanical properties of Cartibeads is determined by a compression test (Young's modulus) that measures the elasticity of the Cartibeads. Stress-strain curves are represented from 3 donors and show an increase of the constraint with larger Cartibeads.

**Figure 2. Amount of GAG in Cartibeads from all donors. a,** Biochemical quantification of GAG content per Cartibead by DMMB assay. **b,** Biochemical quantification of GAG content of Cartibead, normalized to DNA content, expressed in GAG/DNA ratio (µg/µg).

**Figure 3. Comparison of Cartibeads generated in high and low oxygen level and in the 3 and 2-step method. a,** Macroscopic view of the Cartibeads generated with the three-step method under low oxygen conditions for 3 donors. **b,** The safranin O staining of GAG in the Cartibead sections was generated under four different conditions for 1 donor. The Cartibeads were generated by the 3- and 2 step-method under culture conditions with high and low oxygen levels (21% and 5 %). Pictures are representative of 3 donors. Scale bar 200µm. **c,** Evaluation of the quantity of GAG /Cartibead for 3 donors under the 4 different conditions.

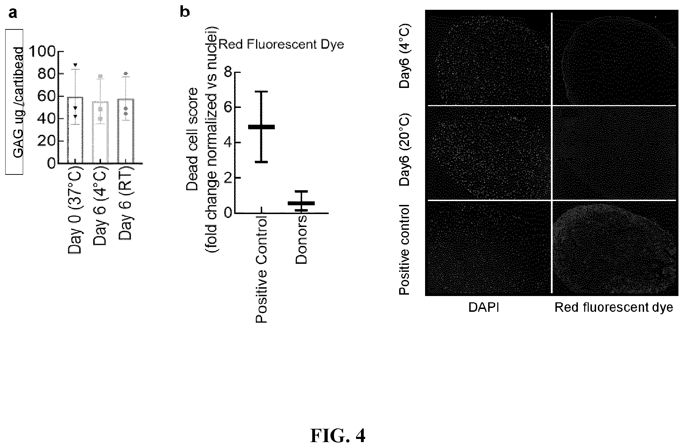

**Figure 4. Stability of Cartibeads. a,** Biochemical quantification of GAG in Cartibeads after completion of the 3-step method and in Cartibeads left for additional 6 days at 37°C (incubator at 5% O2) and at 4°C and Room Temperature (RT) in closed recipients. **b,** The number of dead cells inside the Cartibeads was quantitatively assessed by a red fluorescent dye staining. The positive controls for dead cells were Cartibeads treated previously with 10% of triton before fluorescent staining. Scale bar 100 µm.

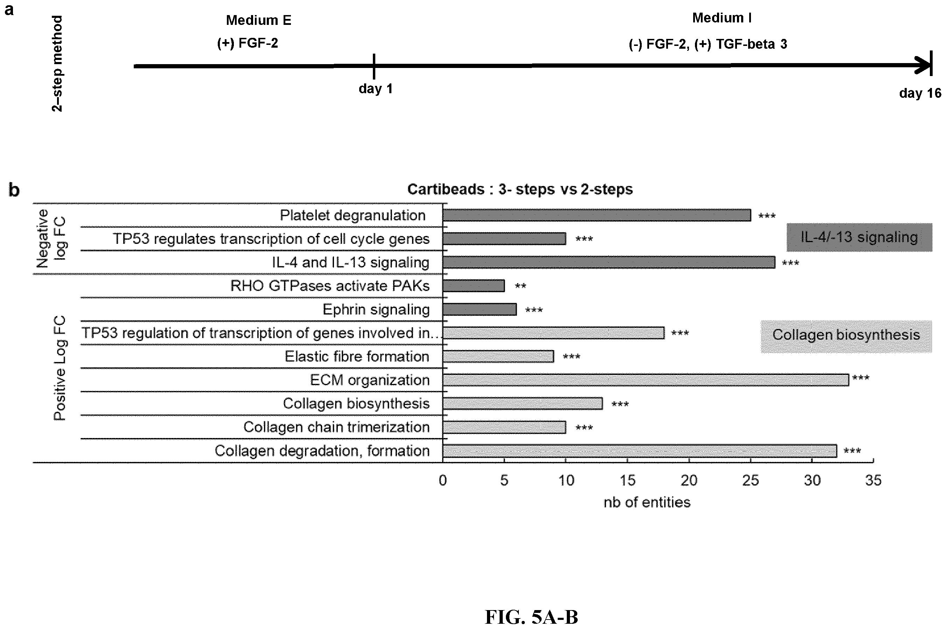

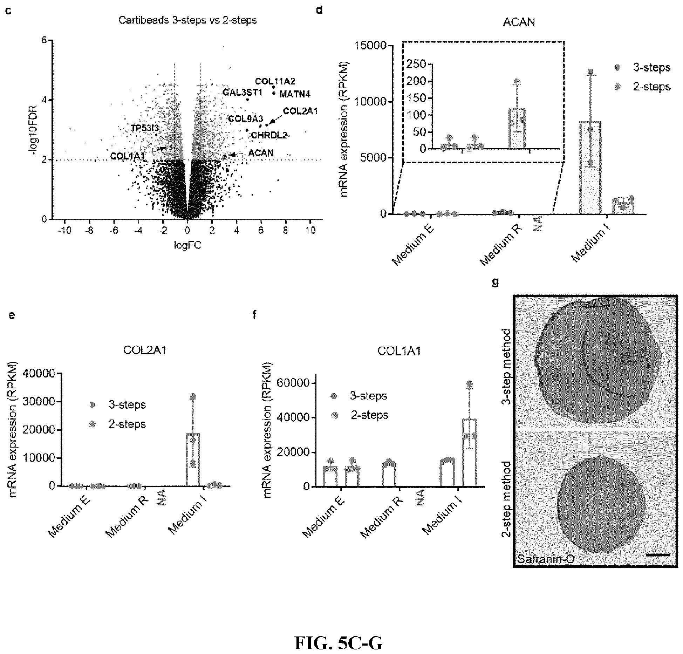

**Figure 5. Improvement of the hyaline characteristics of Cartibeads engineered from the 3-step method compared to the 2-step method. a,** Scheme of the 2-step method for the generation of Cartibeads. Chondrocytes were expanded in step 1 in medium E and directly used in step 2 in medium I for Cartibead generation **b,** Functional annotation clustering of gene set enrichment analysis based on 3-step versus 2-step expression. Histograms show the number of genes for each family of genes for which a significant enrichment was found. In light grey, genes with higher level of expression in the Cartibeads from the 3-step method and in dark grey, genes with lower expression. **c,** Visualization of RNAseq results with Volcano plots that show statistical significance (FDR<0.001) versus magnitude of change (>2-fold change). The Volcano plot highlights genes with fold changes that are statistically significant such as *ACAN, COL2A1*, that are increased in the three-step method compared to the 2-step and *COL1A1* that is decreased. ***p<0.001; **p<0.01 **d, e, f** Data from RNAseq analysis comparing mRNA expression levels (in RPKM) for *ACAN* **(d)**, *COL2A1* **(e)** and *COL1A1* **(f)** genes in the 3-step and 2-step method. NA (Non Applicable) refers to the absence of analysis due to the absence of the step itself **g,** Safranin-O staining of GAG in Cartibeads produced from 3-step method and 2-step method. Scale bar 100μm

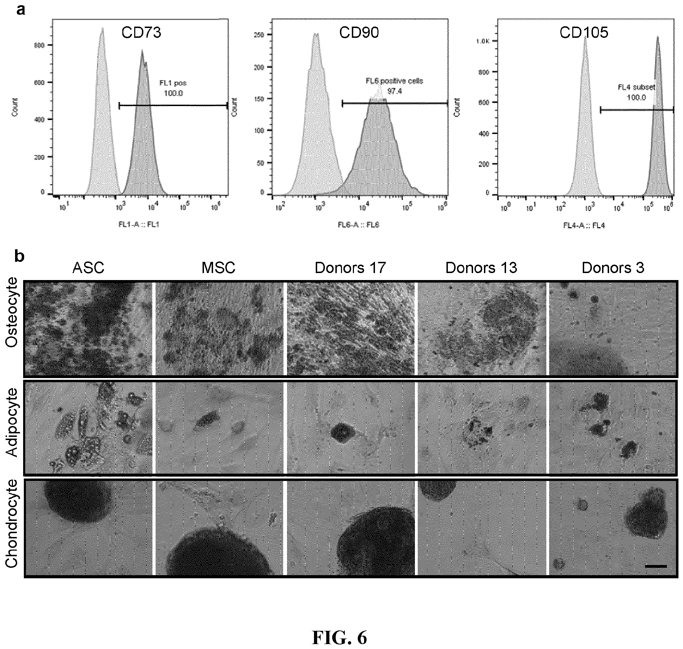

**Figure 6. MSC marker expression. a,** Flow cytometry representing CD73, CD90, and CD105 from left to right respectively. **b,** Differentiation study comparing the Adipose Stem Cells (ASCs), Mesenchymal Stem Cells (MSCs) and chondrocytes potentials of giving osteocytes, adipocytes and chondrocytes. Alizarin Red S was a dye used to stain for calcium deposits, which are indicators of mature osteocytes (top panel), Oil Red O stained neutral lipids characteristics of adipocytes (middle panel) and Safranin-O stained GAG in chondrocytes (low panel). Scale bar 100 μm.

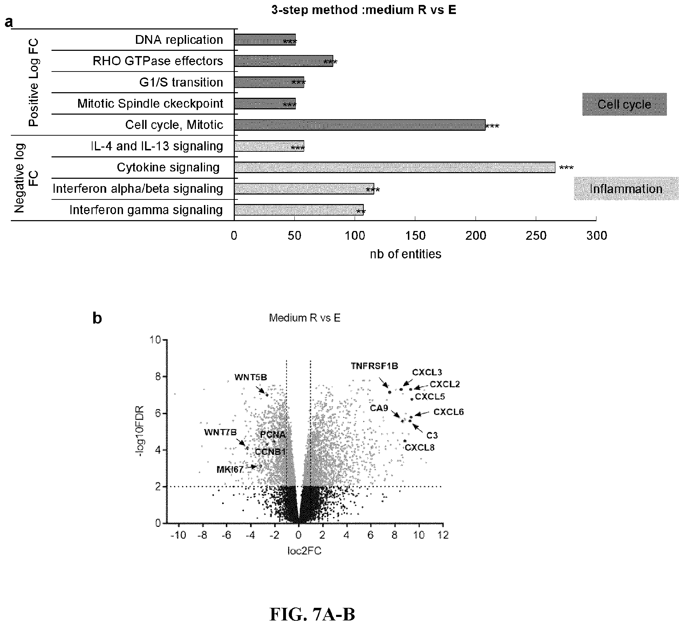

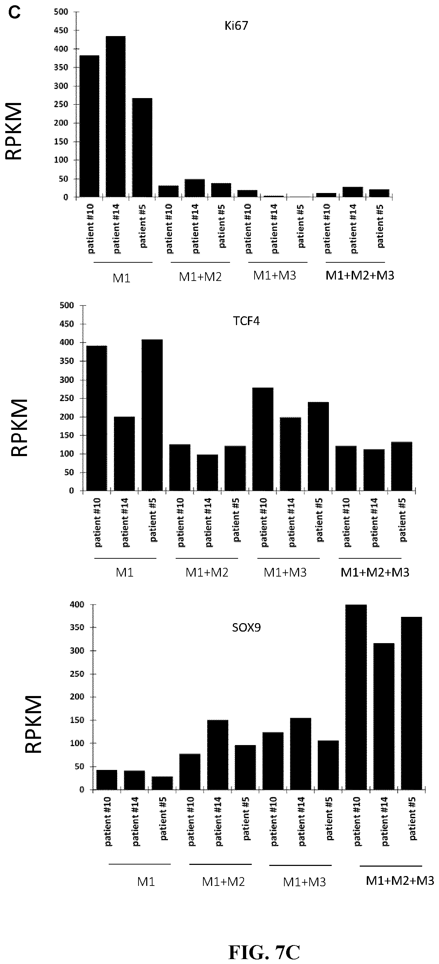

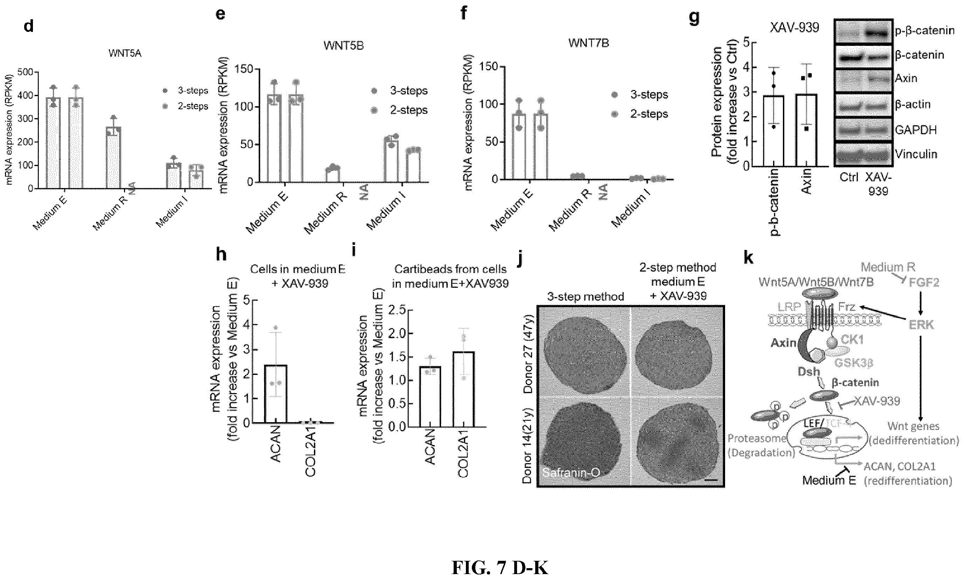

**Figure 7. Inhibition of WNT signaling enhances production of hyaline matrix components, a,** Gene set enrichment analysis and functional grouping based on the differential expression levels of medium R versus medium E in the 3-step method. Graph shows the enrichment for each family of genes (number of genes differentially expressed). ***p<0.001; **p<0.01 **b,** Volcano plot of RNAseq results from chondrocytes in the medium R versus medium E in the 3-step method. c. Data from RNAseq analysis show mRNA expression in RPKM for *ki67* (proliferative marker) (upper panel), *TCF4* (involved downstream the wnt/beta catenin pathway) (middle panel), *SOX9* (transcription factor involved in matrix production) (lower panel). **d, e, f** Data from RNAseq analysis show mRNA expression in RPKM for *WNT5A* (d), *WNT5B* (e), and *WNT7B* (f) in chondrocytes in medium E, R, and I in the 3 and 2-step method. NA (Non Applicable) refers to the absence of analysis due to the absence of the step itself. **g,** Immunoblots show expression of the indicated proteins in presence of the WNT signaling inhibitor XAV-939 (10 μM) in chondrocytes. Histogram shows the densitometry analysis of β-catenin and Axin compared to control. **h, i,** mRNA expression of *ACAN* and *COL2A1* was determined by qPCR in chondrocytes after 4 days in culture in medium E, medium E+XAV-939 and in Cartibeads (2-step method) from chondrocytes cultured in medium E and medium +XAV-839. **j,** Representative image of Safranin-O staining of GAG in Cartibeads generated from chondrocytes cultured in medium R (3-step method) and in medium E +XAV-939 (2-step method). Scale bar 100 μm. **k,** Scheme summarizes the molecular basis for WNT signaling pathway in medium E and WNT inhibition when chondrocytes are cultured in medium R or in medium E+XAV939, allowing the production of a hyaline matrix containing ACAN and type II collagen.

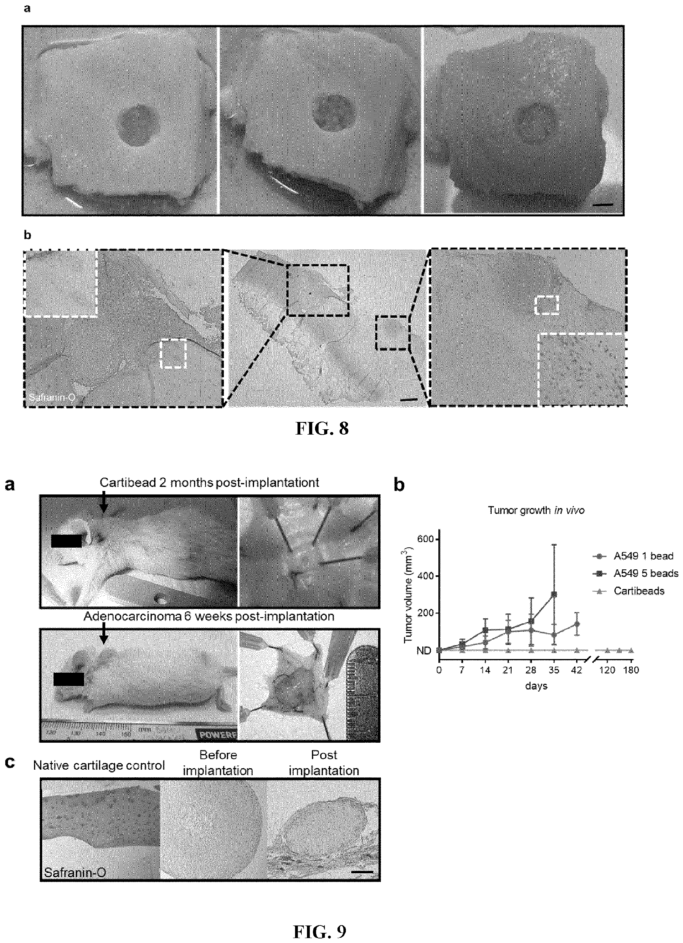

**Figure 8. Cartibeads grafting feasibility in human knee *ex vivo*. a,** Macroscopic view of a distal femur post total knee arthroplasty showing the manually created lesion (left) with surgical instrument, the grafted Cartibeads at day 1 (middle), lesion with the beads 1-month post graft (right) after culture in rotation in medium I. Scale bar 4 mm **b,** Safranin-O staining of GAG of a cross-sectional sample of the *ex vivo* specimen shows that Cartibeads fused together, remain hyalin and integrate with the native cartilage as shown by higher magnification (left and right panel). Data are representative of three independent experiments. Scale bar 1 mm.

**Figure 9. Absence of tumorigenicity of human Cartibeads following graft in immunodeficient mice. a,** Representative picture of a Cartibead ($0.2 \times 10^6$ chondrocytes/cartibead) at 2 months post implantation (top panel) and tumor derived from 5 beads of A549 ($0.2 \times 10^6$ A549 cells/bead) at 6 weeks post graft (lower panel) after subcutaneous graft in the back of mice (black arrows, left panels). **b,** Tumor development after subcutaneous implantation of 1 Cartibead, 1 or 5 beads of A549 cells per SCID mice (n=10 to 14 mice for one Cartibead donor and 2x n=4 mice per A549 beads). **c,** Safranin-O staining of GAG in Cartibeads before (middle panel) and after graft (right panel). Scale bar 200 μm.

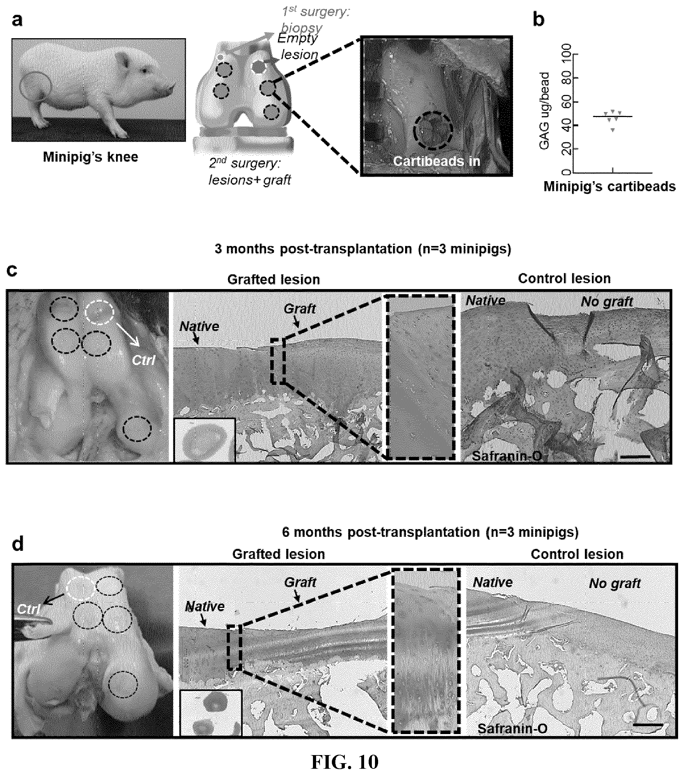

**Figure 10. Integration of hyaline grafts in the knee of minipigs following transplantation of autologous Cartibeads**. **a,** Scheme describes the primary site of the biopsy used for the generation of Cartibeads and the secondary surgery combining the creation of 5 lesions/knee and transplantation of autologous Cartibeads. **b,** Biochemical quantification of GAG (μg/Cartibead) for the 6 minipigs. **c-d,** Macroscopic view (left panel) and Safranin-O staining of GAG of the minipig knee sections (middle panel), the enlarged segments show the tissue remodeling at 3 and 6 months post-transplantation. The transplantation sites are circled in black while the white circle represents the empty lesion as control (right panel). Representative staining for the transplanted lesion (middle panel) and empty lesion (right panel) are shown at three months (c) and six months (d). Scale bar 200 μm.

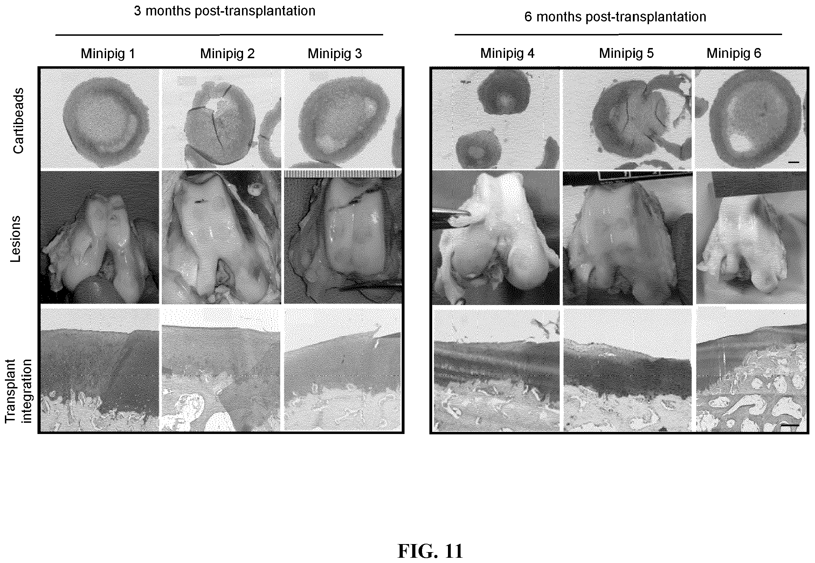

**Figure 11. Pig Cartibead transplantation in minipig generates a hyaline integrated graft. a,** Safranin-O staining of GAG in the Cartibeads before transplantation (upper panel, Scale bar 200 μm) and macroscopic view of the knee (middle panel) and safranin-O staining of GAG in the grafted lesion (low panel, Scale bar 200 μm) for the 6 minipigs used in this study.

## DETAILED DESCRIPTION OF THE INVENTION

The present invention relates to a method for *in vitro* production of cartilage tissue comprising three steps of culture of chondrocytes as described below.

Chondrocytes are originated from mesenchymal cells that have a characteristic phenotype based primarily on the type of extracellular matrix they produce. Precursor cells produce type I collagen, but when they become committed to the chondrocyte lineage, they decrease type I collagen production and start synthesizing type II collagen, which constitutes a substantial protein of the hyaline extracellular matrix. In addition, committed chondrocytes produce proteoglycans which is aggrecan associated to glycosaminoglycans that are highly sulfated.

The term "chondrocyte" as used herein refers to a differentiated cell (committed chondrocyte) obtained from the cartilage with full capacity to synthesize hyaline matrix. The term chondrocyte designates primary cultures (freshly isolated from biopsies) as well as cells expanded *in vitro,* including genetically modified, immortalized, selected, conserved, etc.

### Isolation of subject chondrocytes

Chondrocytes are isolated from biopsies of mature cartilage tissue according to conventional methods, using for example enzymatic digestion of the tissue such as trypsin, chymotrypsin, collagenase, deoxyribonuclease, elastase and hyaluronidase. The cartilage tissue may be a very small piece, preferably less than 5 mm of diameter (equivalent to ~30 mg). Cartilage tissue is preferably mammalian, more preferably human cartilage or equine cartilage. The cartilage tissue may be collected from a healthy donor or a patient, preferably from the knee, ankle, hip, finger, shoulder, more preferably knee or ankle of the patient.

### First step: adherent culture in dedifferentiation medium

The isolated chondrocytes are first cultured on an adherent culture system in a dedifferentiation culture medium.

The adherent culture system suitable to be used in the method according to the invention may be an adherent monolayer culture system or a culture system on feeder cells, preferably an adherent monolayer culture system. The culture system may be in any form suited to the method according to the invention, in particular in the form of a flask, a multi-well plate or a dish.

According to a preferred embodiment, the adherent culture system is an adherent monolayer culture system. This system comprises a solid support, for example glass or plastic, usually coated with a matrix or a substrate promoting cell adherence. The substrate may be a protein substrate consisting of attachment factors and promoting the adhesion of cells to the support. These attachment factors may in particular be selected from poly-L-lysine, collagen, fibronectin, laminin or gelatin.

The matrices that mimic the extracellular matrix and are suitable to be used in the method according to the invention are well-known to the person skilled in the art and many varieties are available commercially. These matrices comprise, for example, matrices of the Matrigel™, Geltrex® type, CELLstart™ or other matrices comprising one or more anchoring proteins such as collagen, laminin, fibronectin, elastin, proteoglycans, aminoglycans or vitronectin. Three-dimensional hydrogel-type matrices may also be used.

According to a preferred embodiment, the matrix is of the Matrigel™ type or CELLstart™ Substrate.

The chondrocytes are cultured on adherent culture system in a medium making it possible to amplify and dedifferentiate the cells in a progenitor/undifferentiated state, named fibroblastic state (fibroblastic-like morphology). In particular, the dedifferentiation medium is a culture medium which allows both the proliferation and the dedifferentiation of chondrocytes into fibroblastic-like cells, also named fibroblastic-like chondrocytes which are dedifferentiated chondrocytes with a morphology of fibroblastic-like cells and which present stem cells characteristics. Preferably, the dedifferentiation medium allows dedifferentiating chondrocytes into fibroblastic-like cells which express mesenchymal stem cell surface markers such as CD105, CD90 and/or CD73. Fibroblastic-like cells are dedifferentiated chondrocytes which have lost the capacity to synthesize hyaline matrix (collagen 2 and GAG). The dedifferentiation medium is a basal medium comprising at least one or more components which allow chondrocytes to amplify and dedifferentiate in an undifferentiated state.

Numerous basal media are available commercially and are well-known to the person skilled in the art. This medium may be a minimum medium particularly comprising mineral salts, amino acids, vitamins and a carbon source essential to cells and a buffer system for regulating pH. The basal medium able to be used in the method according to the invention includes, for example, but are not limited to, DMEM/F12 medium, DMEM medium, RPMI medium, Ham's F12 medium, IMDM medium and KnockOut™ DMEM medium (Life Technologies).

Depending on the medium used, it may be necessary or desirable to add glutamine, vitamin C, one or more antibiotics such as streptomycin, penicillin and/or antimycotic such as Fungizone (amphotericin B).

According to the invention, the dedifferentiation medium activates the Wnt signaling pathway.

In an active state, Wnt ligand binds to frizzled receptors and their co-receptor, lipoprotein-receptor-related protein (LRP) 5/6. This activates dishevelled (DSH) that subsequently inhibits GSK3$\beta$ activity and $\beta$-catenin phosphorylation. $\beta$-catenin is translocated into the nucleus where it interacts with transcription factors TCF/LEF (T-cell specific transcription factor/lymphoid enhancer binding factor). In inactive state, $\beta$-catenin is phosphorylated by glycogen synthase kinase (GSK) 3$\beta$ and the phosphorylated $\beta$-catenin undergoes subsequent ubiquitinylation and proteasomal degradation.

In a preferred embodiment, the dedifferentiation medium is a basal medium which comprises one compound which activates the Wnt signaling, preferably Wnt7B signaling.

The activation of the Wnt signaling pathway may be determined by measuring in dedifferentiated chondrocytes the expression level of wnt RNA, in particular wnt7B, wnt5A or wnt5B, preferably wnt7B RNA and downstream target genes of Wnt signaling pathway such as TCF4. The Wnt signaling pathway is activated in cells when the expression level of the target gene is at least 1.5-fold higher or 2, 3, 4, 5-fold higher than in cells cultured without said compound.

The expression level of mRNA may be determined by any suitable methods known by skilled persons. Usually, these methods comprise measuring the quantity of mRNA. Methods for determining the quantity of mRNA are well-known in the art. For example, the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis), amplification (e.g., RT-PCR) or sequencing (RNA-seq).

The level of the target genes protein may also be determined by any suitable methods known by skilled persons. Usually, these methods comprise contacting a cell sample, preferably a cell lysate, with a binding partner capable of selectively interacting with the target gene protein present in the sample. The binding partner is generally a polyclonal or monoclonal antibodies, preferably monoclonal. The quantity of the protein may be measured, for example, by semi-quantitative Western blots, enzyme-labelled and mediated immunoassays, such as ELISAs, biotin/avidin type assays, radioimmunoassay, immunoelectrophoresis or immunoprecipitation or by protein or antibody arrays.

In particular, the compound capable of activating Wnt signaling pathway can be a Wnt protein that binds to a Wnt receptor or a small molecule GSK-3$\beta$ antagonist, preferably WNT7B, WNT5A or WNT5B, more preferably WNT7B.

Said compound capable of activating Wnt signaling pathway can be selected from the group consisting of: lithium chloride (CAS No. 7447-41-8), CHIR99021 (CAS No. 252917-06-9), SB-216763 (CAS No. 280744-09-4), BIO (6-bromoindirubin-3'-oxime) (CAS No. 667463-62-9).

In a preferred embodiment, said compound is FGF2 (Fibroblast growth factor-2) also known as bFGF or basic form of fibroblast growth factor.

Activation of Wnt signaling pathway allows amplifying and dedifferentiating chondrocyte cells into fibroblastic-like cells. In a preferred embodiment, the dedifferentiation medium comprises at least 5, 10, 15, 20, 30, 40, 50, 60, 70, 80, 90, 100 ng/mL of FGF-2. Preferably, the medium comprises between 5 to 100 ng/mL FGF-2, preferably 20 ng/mL FGF-2.

According to the invention, any molecule, such as polypeptide that acts on chondrocytes in a similar manner as FGF2 molecule in at least one aspect, preferably to amplify chondrocytes in the methods of the invention can be used.

The further components that help chondrocytes to amplify and dedifferentiate the cells in an undifferentiated state are not particularly restricted and may be appropriately selected depending on the purpose. Examples of the component include bone morphogenic proteins (BMP), epidermal growth factor (EGF), platelet-derived growth factors (PDGF), transforming growth factor beta (TGF-$\beta$), insulin growth factor 1 (IGF-1). Each of the other components described above may be used alone, or a plurality of them may be used in combination with a compound capable of activating the Wnt signaling pathway.

In a particular embodiment, the dedifferentiation medium may further comprise one or more growth factors selected from the group consisting of: EGF, TGF-$\beta$ and PDGF-BB and any combination thereof. In this embodiment, EGF, PDGF-BB and TGF-$\beta$ are each present at concentrations in the range of 1 to 100 ng/mL, preferably in the range of 5 to 50 ng/mL. In a more particular embodiment, said TGF-$\beta$ is TGF-$\beta$3.

In a particular embodiment, in methods according to the invention, human chondrocytes are cultured in a dedifferentiation medium comprising FGF-2, PDGF-BB and TGF-$\beta$3, preferably between 5 and 100 ng/mL FGF-2, 1 and 100 ng/mL PDGF-BB and 1 and 100 ng/mL TGF-$\beta$3, more preferably 5 and 50 ng/mL of FGF-2, 5 and 50 ng/mL of PDGF-BB and 5 and 50 ng/mL of TGF-$\beta$3.

In another particular embodiment, in methods according to the invention, equine chondrocytes are cultured in a dedifferentiation medium comprising FGF-2 and EGF, preferably between 5 and 100 ng/mL FGF-2, and 1 and 100 ng/mL EGF, more preferably 5 and 50 ng/mL FGF-2, and 5 and 50ng/mL EGF.

According to a preferred embodiment, the dedifferentiation medium further comprises serum of animal origin. In a preferred embodiment, autologous serum is used. It is also possible to use xenogeneic or allogenic serum. In particular, for culture of human chondrocytes, autologous serum from the human patient or pooled human serum can be used. In a particular embodiment, for equine chondrocytes culture, fetal calf serum can be used. The medium preferably comprises between 2 to 20%, preferably 5 to 15%, more preferably 10 % of serum.

According to a particular embodiment, the chondrocytes are contacted with the dedifferentiation medium for 10 to 20 days, preferably for 10 to 15 days.

The cells are preferably subcultured regularly to prevent the culture from reaching confluence, i.e., from covering the entire available surface. Indeed, confluence induces a cessation of proliferation and unwanted metabolic changes. The cells may be subcultured using standard techniques well-known to the person skilled in the art. They may in particular be detached from the matrix or the support by the action of enzymes such as collagenase IV or by mechanical passage in PBS or any other enzyme-free solution containing EDTA (e.g.: ReleSR, (STEMCELL Technologies)), or by the action of a commercial cell detachment medium such as TrypLE™ Express (Life Technologies), collected by centrifugation, dissociated mechanically and reseeded in a new culture system.

**Second step: adherent culture in redifferentiation medium**

By inactivating Wnt signaling pathway used in the dedifferentiation medium for cell amplification in a second intermediate step of culture, the inventor surprisingly showed that following three-dimensional system culture, the chondrocytes recovered their complete original phenotype and full capacity to synthesize hyaline matrix.

Thus, the method according to the invention further comprises an intermediate step of culturing the fibroblastic-like cells obtained in the first step, in an adherent culture system as described above with a redifferentiation medium that inactivates Wnt signaling pathway, preferably Wnt7B, Wnt5A or Wnt5B, preferably Wnt7B signaling pathway. The chondrocytes are contacted with this redifferentiation medium to reverse the fibroblastic phenotype and redifferentiate fibroblastic-like cells to chondrocytes with full capacity to synthesize hyaline matrix. In this second step, cells change their morphology and become less elongated, larger, with apparent granular endoplasmic reticulum in the cytoplasm under optical microscopy, signaling thus a high level of protein synthesis activity. Chondrocytes start to re-express aggrecan, a proteoglycan associated with GAG. Collagen II is not re-expressed at the end of the second step.

Preferably, the contacting is carried out by simply changing the culture medium. Alternatively, it may be carried out by subculturing in an adherent culture system as described above comprising the reversion medium.

According to an embodiment, the adherent culture system is an adherent monolayer culture system as described above. According to a preferred embodiment, the matrix is of the Matrigel™ type or CELLstart™ Substrate.

In a first embodiment, the redifferentiation medium can be a redifferentiation medium comprising basal medium in which compound that activates Wnt signaling pathway as described previously is removed.

By removing a compound, it is intended that said redifferentiation medium comprises compound at a concentration that does not allow activation of the Wnt signaling pathway.

In more preferred embodiment, said redifferentiation medium is FGF-2 free redifferentiation medium. Said FGF-2 free redifferentiation medium comprises a basal medium comprising less than 0.5 ng/mL of FGF-2, preferably less than 0.4, 0.3, 0.2, 0.1 ng/mL of FGF-2.

In another embodiment, said redifferentiation medium is a basal medium comprising an inhibitor of the Wnt signaling pathway.

Said inhibitor of the Wnt signaling pathway can be a small molecule selected from the group consisting of: compounds that target Dvl protein such as NSC668036 (CAS No. 144678-63-7), 3289-8625 (CAS No. 294891-81-9), J01-017a, TMEM88, KY-02061, KY-02327, BMD4702 (CAS No. 335206-54-7), Niclosamide (CAS No. 50-65-7), DK-520, Sulindac (CAS No. 38194-50-2); compounds that target β-catenin destruction complex such as Pyrvinium (CAS No. 7187-62-4); natural compounds such as derricin (CAS No. 34211-25-1), derricidin (CAS No. 38965-74-1), carnosic acid (CAS No. 3650-09-7); compounds that target TCF/LEF transcription reporter such as ICG-001 (CAS No. 847591-62-2), PNU-74654 (CAS No. 113906-27-7), Windorphen (CAS No. 19881-70-0); compounds that target Pren such as IWP-L6 (CAS No. 1427782-89-5), Wnt-C59 (CAS No. 1243243-89-1), LGK974 (CAS No. 1243244-14-5), ETC-159 (CAS No. 1638250-96-0), compounds that target TNKS such as XAV939 (CAS No. 284028-89-3), E7449 (CAS No. 1140964-99-3), preferably XAV939.

In another embodiment, small inhibitory RNAs (siRNAs) can also be used to decrease gene expression level of at least one protein involved in Wnt pathway signaling. In a preferred embodiment, said Wnt signaling protein gene expression can be reduced by introducing into a cell a small double stranded RNA (dsRNA), or a vector or construct causing the production of a small double stranded RNA, such that Wnt signaling pathway is inactivated (i.e., RNA interference or RNAi). Methods for selecting an appropriate dsRNA or dsRNA-encoding vector are well-known in the art for genes whose sequence is known (e.g., see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

In another embodiment, short hairpin RNA (shRNA) can also be used to decrease the gene expression level of Wnt signaling protein. A short hairpin RNA (shRNA) is a sequence of RNA that makes a tight hairpin turn that can be used to silence target gene expression via RNA interference (RNAi). Expression of shRNA in cells is typically accomplished by delivery of plasmids or through viral or bacterial vectors. The promoter choice is essential to achieve robust shRNA expression. At first, polymerase III promoters such as U6 and HI were used; however, these promoters lack spatial and temporal control. As such, there has been a shift to using polymerase II promoters to regulate expression of shRNA.

In a preferred embodiment, siRNA is used to decrease gene expression level of Wnt5B, Wnt7B, Wnt5A or β-catenin, preferably Wnt7B.

Basal culture medium may be a minimum medium particularly comprising mineral salts, amino acids, vitamins and a carbon source essential to cells and a buffer system for regulating pH. The basal medium able to be used in the method according to the invention includes, for example, but are not limited to, DMEM/F12 medium, DMEM medium, RPMI medium, Ham's F12 medium, IMDM medium and KnockOut™ DMEM medium (Life Technologies).

In a particular embodiment, the redifferentiation medium is an FGF-2 free redifferentiation medium which comprises a basal medium comprising less than 0.5 ng/mL FGF-2 and comprises at least one or more components which promote fibroblastic-like cells to redifferentiate into chondrocytes.

In a particular embodiment, the redifferentiation medium comprises transformation growth factor β (TGFβ), more preferably TGF-β3. Preferably, the redifferentiation medium comprises between 1 and 100 ng/mL TGF-β3, more preferably between 5 and 50 ng/mL TGF-β3.

In a more particular embodiment, the redifferentiation medium further comprises FGF-7, also known as keratinocyte growth factor. FGF-7 participates in cell proliferation and in cell differentiation. Preferably, the redifferentiation medium comprises between 1 and 100 ng/mL FGF-7, more preferably between 5 and 50 ng/mL FGF-7.

According to a particular embodiment, the redifferentiation medium used further comprises serum of animal origin as described above.

In a preferred embodiment, said TGF-β, FGF-7 and/or serum are replaced by platelet lysate. Platelet lysate is a growth factor-rich cell culture supplement derived from blood platelets after freeze/thaw cycles. The freeze/thaw cycle causes the platelets to lyse, releasing a large quantity of growth factors necessary for cell expansion. In a preferred embodiment, the redifferentiation medium comprises between 5 to 20%, preferably 10% of platelet lysate. In a preferred embodiment, said platelet lysate is a pooled human platelet lysate.

The components that may affect chondrocyte differentiation are not particularly restricted and may be appropriately selected depending on the purpose. Examples of the component include insulin, insulin-like growth factors (IGF-1), transformation growth factor β, bone morphogenic proteins (BMP), selenium, transferrin, ethanolamine, platelet-derived growth factors. Each of the other components described above may be used alone, or a plurality of them may be used in combination.

According to a particular embodiment, the chondrocytes are contacted with the redifferentiation medium for 2 to 10 days, preferably for 3 to 7 days, more preferably 7 days.

The cells may be subcultured regularly to prevent the culture from reaching confluence, i.e., from covering the entire available surface. Indeed, confluence induces a cessation of proliferation and unwanted metabolic changes. The cells may be subcultured using standard techniques well-known to the person skilled in the art as described above.

**Third step: 3D-culture system in Induction/maturation medium**

To allow cartilage tissue formation, the chondrocytes obtained in second step are then cultured on a three-dimensional culture system in a maturation medium also named induction medium. In this step, the chondrocytes produce their own extracellular matrix to form cartilage tissue, in particular they start synthesizing collagen of type II. The cartilage tissue obtained is a three-dimensional tissue of varying size, which can be referred as spheroid. Said spheroid is composed of cells contained in the spheroid and of a hyaline matrix formed by these cells.

The maturation medium is a basal medium comprising at least one or more components which allow chondrocytes to produce hyaline matrix and form cartilage tissue.

In particular, the maturation medium inactivates Wnt signaling pathway. In an embodiment, the maturation medium comprises basal medium in which compound that activates Wnt signaling pathway as described previously is removed. In another embodiment, said maturation medium is a basal medium comprising an inhibitor of the Wnt signaling pathway as previously described.

In a preferred embodiment, said maturation medium is a basal medium in which FGF-2 growth factor is removed. In other terms, the maturation medium is a basal medium which comprises less than 0.5 ng/mL of FGF-2, preferably less than 0.4, 0.3, 0.2, 0.1 ng/mL of FGF-2.

The components that may affect chondrocyte maturation are not particularly restricted and may be appropriately selected depending on the purpose. Examples of the component include TGF-β growth factors, insulin, insulin-like growth factors (IGF-1), transformation growth factor β, bone morphogenic proteins (BMP), selenium, transferrin, ethanolamine, epidermal growth factor, platelet-derived growth factors. Each of the other components described above may be used alone, or a plurality of them may be used in combination.

According to a particular embodiment, the maturation medium is a basal culture medium as described above further comprising TGF-β, preferably TGF-β3.

Preferably, the maturation medium comprises between 1 and 100 ng/mL TGF-β3, more preferably between 5 to 50 ng/mL TGF-β3.

According to an embodiment, the chondrocyte maturation medium is a basal culture medium comprising TGF-β3, IGF-1, BMP-2 and insulin. Preferably, the chondrocyte maturation medium comprises TGF-β3, insulin, IGF-1, BMP-2, selenium, transferrin and ethanolamine, more preferably TGF-β3, insulin, selenium, transferrin and ethanolamine.

Preferably, the maturation medium comprises between 1 and 100 ng/mL TGF-β3, preferably 5 and 50 ng/mL TGF-β3, between 1 and 100 ng/mL IGF-1, preferably 5 and 50 ng/mL IGF-1, and/or between 1 and 100 ng/mL preferably 5 and 50 ng/mL BMP-2.

According to a particular embodiment, the chondrocytes are cultured on three-dimensional culture system in a maturation medium for 10 to 20 days, preferably for 10 to 15 days.

The 3D culture enables cells to connect to each other in order to favour extracellular matrix synthesis by chondrocytes. The 3D culture system can be static or dynamic. Static method implies providing cells a modality to form aggregates due to static physical forces. The static methods include but are not limited to hanging drop method, culture in liquid on non-adherent substrate such as a thin coating of agar or agarose, culture on low attachment surface plates. Dynamic methods imply forced cellular aggregation. The dynamic methods include but are not limited to spinner flask culture, the rotating wall vessel and pellet culture. In a preferred embodiment, the 3D culture system is a pellet culture. In the pellet culture, the chondrocytes are dispensed in plates and centrifuged to aggregate cells and form a pellet.

In a preferred embodiment the 3D culture in maturation medium is performed in hypoxia atmosphere to improve cartilage formation. In a preferred embodiment, chondrocytes are cultured in 3D culture system in a maturation medium in an atmosphere of less than 10% (v/v) O2, more preferably less than 7% O2, more preferably at 5% O2.

The cells are maintained in the chondrocyte maturation medium until obtaining cartilage tissue. As used herein, the cartilage refers to hyaline cartilage. During this period, and in a conventional manner, the culture medium may be changed regularly, preferably every 2 or 3 days.

The quality of the cartilage tissue obtained can be tested by measuring the content of glycosaminoglycan (GAG) and collagen II in engineered cartilage tissue. The quality and quantity of the cartilage tissue may also be determined by measuring the quantity of GAG/microtissue or by GAG/double strand DNA ratio. The presence of GAG, collagen or double strand DNA can be evaluated by any methods known in the art. For example, GAG can be revealed by safranin-O coloration or more quantitatively by a dosage of GAG using dimethylmethylene blue assay (DMMB). Collagen II can be evaluated by immuno-staining or by a more quantitative assay such as ELISA.

Thus, optionally, the method according to the invention may comprise an additional step consisting of measuring or evaluating the presence of GAG and/or collagen II in engineered cartilage tissue.

**Engineered cartilage tissue (Cartibeads)**

The present invention also relates to the engineered cartilage tissue obtainable by the method according to the invention. According to the invention, said engineered cartilage tissue is in a form of spheroid presenting a glycosaminoglycan (GAG) content per spheroid between 10 and 100 μg/spheroid.

The present invention relates to engineered cartilage tissue in a form of spheroid wherein said spheroid presents GAG content per spheroid of at least 15, 16, 17, 18, 19 or 20 μg/spheroid, between 10 and 100 μg/spheroid, more preferably between 15 and 60 μg/spheroid.

The present invention relates to engineered cartilage tissue in a form of spheroid wherein said spheroid presents a GAG/double strand DNA ratio of at least 10, 15 or 20, preferably between 10 to 100, more preferably between 10 to 80.

Said spheroid of cartilage tissue has a diameter of 1 to 2 mm and comprises between 50 000 to 250 000 cells, preferentially 200 000 cells.

**Pharmaceutical composition**

In another aspect, the present invention also relates to a pharmaceutical composition comprising the engineered cartilage tissue of the invention, and one or more pharmaceutically acceptable excipients.

The pharmaceutically acceptable excipients must be compatible with the cells and may be, for example, a culture medium, a buffer solution or a saline solution.

In a preferred embodiment, the pharmaceutical composition is suitable for parenteral administration, preferably by subcutaneous route, in particular for administration directly in cartilage or bone tissue. The pharmaceutical composition may be formulated in accordance with the standard pharmaceutical practices known to the person skilled in the art.

In a particular embodiment, the pharmaceutical composition comprises the cartilage tissue of the invention, encapsulated in a biocompatible matrix.

The pharmaceutical composition may also comprise one or more additional active compounds, for example compounds known to improve cell survival or proliferation or to prevent contamination.

**Therapeutic application**

According to still another aspect, the present invention relates to the engineered cartilage tissue of the invention or the pharmaceutical composition comprising said engineered cartilage tissue for therapeutic use, in particular for the treatment of cartilage defects and cartilage degenerative diseases in a subject in need thereof.

The present invention thus relates to the engineered cartilage tissue of the invention for use in the treatment of cartilage defects and cartilage degenerative disease in a subject in need thereof. It also relates to a pharmaceutical composition according to the invention for use in the treatment of cartilage defects and cartilage degenerative disease in a subject in need thereof.

The present invention is particularly suitable for autologous cartilage tissue transplantation in a subject in need thereof. In transplantation of autologous engineered cartilage tissue of the invention, the chondrocytes are first isolated from said subject, preferably from biopsy of mature cartilage tissue of said subject. The chondrocytes are then cultured according to the method of the invention to obtain autologous engineered cartilage tissue and used for the treatment of cartilage defects and cartilage degenerative diseases in said patient.

Thus, in a preferred embodiment, the present invention relates to the use of autologous engineered cartilage tissue of the invention or pharmaceutical composition comprising said autologous engineered cartilage tissue for the treatment of cartilage defects and cartilage degenerative diseases in a subject in need thereof.

In another embodiment, allogenic transplantation can be used in a subject in need thereof.

As used herein, the terms "cartilage defects and cartilage degenerative disease" include but are not limited to cartilage lesions, arthrosis, rheumatism or osteoarthritis.

In a particular embodiment, the engineered cartilage tissue of the invention or pharmaceutical composition thereof is indicated for patients affected by a focal articular cartilage lesion or early osteoarthritis, in order to delay or avoid total knee replacement not commonly proposed for patients under the age of 60 due to the limited lifetime of prosthesis. Thus, in a preferred embodiment, the cartilage defects and cartilage degenerative disease is a focal articular cartilage lesion or early osteoarthritis.

The term "treatment" as used in this document refers to an improvement or disappearance of symptoms, such as pain and mobility decrease, a slowing of the progression of the disease, a cessation of the evolution of the disease or a disappearance of the disease. This term also includes both preventive and curative treatment.

As used here, the term "subject" or "patient" as used herein refers to mammals. Mammalian species that can benefit from the disclosed methods of treatment include, but are not limited to, humans, non-human primates such as apes, chimpanzees, monkeys, and orangutans, domesticated animals, including dogs and cats, as well as livestock such as horses, cattle, pigs, sheep, and goats, or other mammalian species including, without limitation, camels, mice, rats, guinea pigs, rabbits, hamsters, and the like. In particular embodiment, said subject is human or horse.

The term "therapeutically effective amount" as used herein refers to a sufficient amount to decrease pain symptoms or increase mobility of a subject presenting cartilage defects and/or cartilage degenerative diseases.

In a preferred embodiment, 10 to 50 spheroids of the cartilage tissue of approximately 1 to 2 mm of diameter are to be administered per $cm^2$ of lesion to completely cover it.

Spheroids self-adhere to the subchondral bone and the internal edge of the chondral lesion.

Engineered cartilage tissue or the pharmaceutical composition of the invention may be administered by grafting directly said cartilage onto the surface of the cartilage or the supporting matrix or into the local environment of the cartilage or the supporting matrix of the subject.

The engineered cartilage tissue or the pharmaceutical composition of the invention can be administered to the subject by an open-joint surgery procedure or through arthroscopy, preferably through arthroscopy to allow faster recovery of patients.

Once transplanted *in vivo,* chondrocytes inside the cartilage tissue responds to mechanical loads by continuing to produce matrix in order to fill the full-thickness defect till the surface of the damaged tissue and have the potential to fuse altogether *in vivo* to form a homogeneous cartilage tissue.

**Kit**

Also disclosed is a kit for *in vitro* production of cartilage tissue. This kit comprises:

- a first container containing one or more compounds present in the dedifferentiation medium as described above, preferably FGF-2, more preferably FGF-2 and a growth factor selected from the group consisting of EGF, TGF-β3 and PDGF-BB and any combination thereof,
- a second container containing one or more compounds present in the redifferentiation medium as described above, preferably TGFβ3, more preferably TGFβ3 and FGF-7, said TGF-β3 and/or FGF-7 can be replaced by platelet lysate, and
- a third container containing one or more compounds present in the maturation medium as described above, preferably TGF-β3, more preferably TGF-β3 and one compound selected from the group consisting of: IGF-1, BMP-2, insulin, selenium, transferrin and ethanolamine and any combination thereof, again more preferably TGF-β3 and insulin, selenium, transferrin and ethanolamine.

Preferably, the kit comprises containers each comprising one or more compounds at a concentration or in an amount that facilitates the reconstitution and/or the use of the differentiation and/or maturation medium and the implementation of the method according to the invention. The kit may also comprise a container containing a basal medium as described above.

The kit may also comprise an adherent culture system, in particular in the form of a flask, a multiwell plate or a dish.

The kit may also comprise instructions indicating the methods for preparing and/or using the differentiation or maturation media for *in vitro* production of cartilage tissue according to the method of the invention.

Also disclosed is the use of the kit according to the invention for *in vitro* production of chondrocytes according to the methods of the invention.

**Method for screening molecules of therapeutic interest.**

Also disclosed is the use of the cartilage tissue of the invention for screening molecules of therapeutic interest.

The molecules of therapeutic interest may be in particular molecules inhibiting the degenerative process in cartilage degenerative disease. These molecules may be particularly usable in the treatment or the prevention of cartilage defects or cartilage degenerative diseases as described above.

Also disclosed is a method for screening molecules of interest comprising

i) contacting the cartilage tissue of the invention with candidate molecules, and
ii) selecting the molecules having the desired activity.

The present invention relates in particular to a method for screening molecules that inhibits the cartilage degenerative process comprising:

i) contacting the cartilage of the invention with one or more candidate molecules, and
ii) selecting the molecules that inhibits cartilage degenerative process.

The production of cartilage tissue may be evaluated by techniques well-known to the person skilled in the art, such as, for example, methods comprising measuring GAG or collagen II.

Depending on the nature of the molecules sought, the chondrocytes used to produce the cartilage tissue may be obtained from a healthy subject or a subject having a cartilage defect or cartilage degenerative disease as defined above.

Other features and advantages of the invention will become more apparent upon reading the following examples given by way of non-limiting illustration.

**EXAMPLES**

**MATERIAL AND METHODS**

**Chemicals**

XAV939 was obtained by Sigma and used at the concentration of 10 μM for 4 days in 2D culture in medium E.

**Production of Cartibeads**

The following media described in the Table 1 have been used in the examples.

**Table 1: Composition of the media used in examples.**

| Medium | Origin of chondrocytes | Composition |
|---|---|---|
| Dedifferentiation medium (medium 1, also named medium E) | Human chondrocytes | -DMEM 4.5g/l of glucose<br>-Glutamax<br>-non-essential amino acids<br>-10% of pooled human serum<br>-20 ng/ml FGF-2<br>-10 ng/ml PDGF-BB<br>-5 ng/ml TGF beta 3; or<br>-DMEM 4.5g/l of glucose<br>-Glutamax<br>-non-essential amino acids<br>-10% of pooled human serum<br>-20 ng/ml FGF-2 |
| | Equine chondrocytes | -DMEM 4.5g/l of glucose<br>-Glutamax<br>-non essential amino acids<br>-10% of fetal calf serum<br>-20 ng/ml FGF-2<br>-20 ng/ml EGF. |
| Redifferentiation medium (also named chondrogenic commitment medium) (medium 2, also named medium R) | Human chondrocytes | -DMEM 4.5g/l of glucose<br>-Glutamax<br>-non-essential amino acids<br>-10% of pooled human platelet lysate |
| | Equine chondrocytes | -DMEM 4.5g/l of glucose<br>-Glutamax<br>-Non-essential amino acids<br>-10% of fetal calf serum |

(continued)

| Medium | Origin of chondrocytes | Composition |
|---|---|---|
| Maturation medium (medium 3, also named medium I) | Human chondrocytes | -DMEM 4.5g/l of glucose<br>-Glutamax<br>-Non-essential amino acids<br>-ITS-X (insulin, selenium, transferrin, ethanolamine) 1x<br>-10-100 ng/ml TGF beta 3<br>-10-100 ng/mL IGF<br>- 10-100 ng/ml BMP-2 or<br>-DMEM 4.5g/l of glucose<br>-Glutamax<br>-Non-essential amino acids<br>-ITS-X (insulin, selenium, transferrin, ethanolamine) 1x<br>-10-100 ng/ml TGF beta 3 |
| | Equine chondrocytes | -DMEM 4.5g/l of glucose<br>-Glutamax<br>-Non-essential amino acids<br>-ITS-X (insulin, selenium, transferrin, ethanolamine) 1x<br>-10-100 ng/ml TGF beta 3<br>-10-100 ng/mL IGF I<br>-10-100 ng/ml BMP-2 |

Sample Collection

Human cartilage samples were obtained from life consenting donors (age 18 to 80 years old), post orthopedic procedures for various indications (Table 2).

**Table 2: Characteristics of the donors including sex, age and pathology.**

| ID | Sex | Age | Pathology |
|---|---|---|---|
| Donor 1 | M | 21 | Trauma |
| Donor 2 | M | 47 | Trauma |
| Donor 3 | M | 29 | Trauma |
| Donor 8 | F | 65 | Knee arthrosis |
| Donor 9 | M | 80 | Knee arthrosis |
| Donor 13 | F | 64 | Knee arthrosis |
| Donor 14 | M | 69 | Knee arthrosis |
| Donor 15 | M | 87 | femoral condyl necrosis |
| Donor 17 | F | 28 | Trauma (pattelar dislocation) |
| Donor 18 | M | 58 | Degenerative focal lesion |
| Donor 19 | F | 60 | Knee arthrosis |
| Donor 20 | M | 38 | Osteochondrite dissccance |
| Donor 21 | M | 68 | Knee arthrosis |
| Donor 23 | M | 63 | Knee arthrosis |

(continued)

| ID | Sex | Age | Pathology |
|---|---|---|---|
| Donor 24 | F | 74 | Knee arthrosis |
| Donor 27 | M | 82 | Knee arthrosis |
| Donor 31 | M | 76 | talus (Ankle arthrosis) |
| Donor 37 | F | 70 | talus (Ankle arthrosis) |
| Donor 39 | M | 74 | talus (Ankle arthrosis) |

The collected cartilage was transferred to the laboratory in a sterile recipient in normal saline (NaCl 0.9%) at room temperature. Human cartilage samples collection was approved by the Swiss Ethics Committee (BASEC, 2016-00656).

Minipig's cartilages were obtained from the lateral trochlear holder, (~30 mg), harvested from the right knee of the minipigs.

The cartilage (~ 30 mg) was sliced into small pieces (1 mm) to facilitate the extraction of chondrocytes by enzymatic digestion, then placed in rotation at 37 °C overnight, with collagenase of type II (400 U/ml, ThermoFisher) in the medium E containing antibiotic (gentamicin, 50 ug/ml) and antifungal (amphotericin B or fungizone®, 0.250 ug/ml).

Cell culture and production of Cartibeads

Cells were washed and plated (p0) onto extracellular matrix (MaxGel™, Sigma) pre-coated T25 cm$^2$ flasks, then cultured for 12 to 16 days in medium E with gentamicin and fungizone® which were removed after 5 days of cell expansion. All the 2D cell cultures were conducted on extracellular matrix-coated flasks. At confluence, cells were passaged in 1 T75 (p1) and split later in 2x T75 (p2) to reach confluence. At this stage cells can be frozen for back-up (p3). After cell expansion in medium E (step 1), cells were cultured for 7 days in medium R, in step 2, where a decrease of their growth was observed before proceeding to 3D culture in step 3. In step 3, chondrocytes were collected and aggregated in medium I to obtain $0.2 \times 10^6$ cells/well in conical 96 well plates (~$20 \times 10^6$ cells/plate). The 96 well plates were centrifugated 5 min at 300g to allow cell aggregation and formation of Cartibeads after 15 days in 3D culture. Cartibeads were obtained from chondrocytes up to passage 8. These beads were removed from the 96 well plates and pooled together and can be maintained in medium **I** at 4 to 23°C (room temperature) for up to 6 days with a high stability.

**GAG quantification**

Glycosaminoglycan (GAG) content was evaluated by the dimethylmethylene blue assay (DMMB) (Sigma, 341088). Chondroitin sulphate A (Sigma, C9819) was used to generate 6 standards, concentrations ranging from 0 to 50 μg/mL. Chondroitin sulphate C (Sigma, C4384) was used to generate a low and high Internal Quality Control (IQC), concentrations 15 and 35 μg/mL, respectively. Cartibeads were digested with proteinase K (1mg/ml) (Promega, V3021) in Tris-HCI, 50 mM, pH 8 (Sigma), for $15 \pm 2$ hours at 56°C. The enzymatic digestion was stopped by incubation at 97°C for 15 minutes. The resulting sample was then diluted (1/5-1/10) in Tris-HCI, 50 mM, pH 8 for the assay. 100μL of standards, ICQ's and samples were read with a spectrophotometer in triplicates ($\lambda$ = 525 nm) after 5 minutes reaction with 1mL DMMB solution. Then, the GAG content was normalized to the DNA amount, which was measured with PicoGreen-Qubit assay. Standards and ICQs were prepared from calf thymus DNA in two separate preparations. The standards and ICQI were prepared in 200 mM Tris-HCI, 20 mM EDTA, pH 7.5, (TE) buffer; the samples are those from the proteinase K digestion, then diluted 1 /15 in TE buffer. For this assay, 100μL of standard, IQC, and then sample were taken and divided into triplicates. Then 100μL of 1/200 diluted PicoGreen Quant-It® (ThermoFisher, P11496) was added. The sample was then incubated for 5 minutes, during which time the intercalant is complexed with the DNA. Finally, the reading was performed in Qubit 4 Fluorometer (ThermoFisher, Q33238), with excitation peak at 485 nm.

**Cell viability of Cartibeads**

Samples were tested for viability through an assay using a red fluorescent dye, Zombie Aqua™ (Biolegend, 423101) which is an amine-reactive fluorescent dye that is non-permeant to living cells but permeant to cells with compromised membranes, thus, allowing to assess living versus dead cells. Three-D samples were collected by the end of step 3, then washed with PBS then Zombie dye diluted in PBS (1:100) was added for 20 minutes, samples were kept in the dark. Samples were subsequently cut through cryostat microtome at 3 μm of thickness and mounted on superfrost plus slides. Afterwards they were fixed with formol 4% and then treated with Hoechst (Molecular probe, H3570Thermofisher) diluted in PBS (1:2000) for 10 minutes. Control of the assay was achieved by adding Triton™ (Sigma, X100-100ML) at 10% in PBS, for 1 hour at room temperature (RT) before starting the Zombie dye step.

**Immunohistochemical staining**

For immunohistochemical staining of formalin-fixed paraffin-embedded cartilage tissues and Cartibeads, the paraffin blocks containing the samples were cut with the microtome at $5\mu m$. The slides were dried overnight at 47°C. Slides were deparaffinated by Xylol and rehydrated by consecutive alcohol baths (concentrations 100%, 95%, and 70%). Two techniques of unmasking were used. For native cartilage coming from minipigs, the inventors used a 20mg/mL solution of hyaluronidase in 0.1M phosphate buffer, placed on slides for 1 hour at 37°C. Slides were rinsed in PBS twice for 5 minutes to remove hyaluronidase. Cartibeads samples were immersed in 0.01M citrate buffer bath at pH=6, heated 3 times, 5 minutes each in microwave at 620w and then cooled in ice bath for 20 minutes. The slides were then rinsed in PBS for 5 minutes. The primary antibodies were then used (Collagen I Abcam, ab6308; Collagen II Abcam ab85266 and ThermoFisher MAS-12789). Of note, the two type II collagen antibodies that were used showed similar results. The antibodies were placed on different samples, diluted 1:100 in 0.3% triton:PBS for 1 hour. After rinsing for 5 minutes with PBS, a secondary antibody was used, a biotin-conjugated anti-mouse (Vector lab, BA-2000), anti-rabbit IgG (Vector lab BA-1000), and an avidin-biotin peroxidase detection system with 3,3'-diaminobenzidine substrate (Vector Labs). Samples were counterstained with hematoxylin. Dehydration was achieved by vigorously dehydrate by shaking the rack 10-20 times in alcohol baths (concentration 95% and 100%), followed by a Xylol bath until slides assembly with Eukitt resin (Batch A1113, KiNDO1500). A Nikon Eclipse C1 Confocal microscope as well as a Nikon Eclipse TE2000-E were used for imaging.

**Safranin-O staining**

Safranin-O staining of cartilage spheres and native cartilage formalin-fixed paraffin-embedded samples was performed, in order to reveal glycosaminoglycans (GAG) on $5\mu m$ paraffin slices. Slides were deparaffinated by Xylol and rehydrated by consecutive alcohol bath (concentration 100%, 95% and 70%) with a final 5-minute distilled water bath. Then, hematoxylin staining was used for nucleus counterstaining, followed by a 5-minute wash with running hot water. Fast Green (Sigma F7252) was used to stain the cytoplasm, then washed out with acetic acid. The slides were washed out immediately with distilled water. Safranin-O 0.1% in water bath (Sigma S2255) was applied for staining of GAGs for two and a half minutes, then washed repeatedly with distilled water. Dehydration was achieved by vigorously shaking the rack 10-20 times in alcohol baths (concentration 95% and 100%), followed by a Xylol bath until slides assembly with Eukitt resin (Batch A1113, KiNDO1500).

**Multipotency assessment**

Monolayer-expanded human MSCs/ASCs/chondrocytes were differentiated into the chondrogenic, adipogenic, and osteogenic fate to assess their multipotency. The osteogenic differentiation was assessed using Alizarin Red S (Merck, TMS-008-C) staining. The adipogenic differentiation was assessed using Oil Red O (Sigma, 01391) staining and the chondrogenic was assessed using Safranin-O (Sigma, S2255) /Fast Green staining (Sigma F7252).

**Biomechanics measurements**

Compression tests are performed with MTS criterion pull-push machine (model 42) equipped with a 1N load cell on cartilage beads ranging from 0.68 mm to 1.54 mm in diameter and in native cartilage. Compression speed was set at 0.01 mm.s$^{-1}$ with a maximum load compression imposed at 0.2N; maximum ten beads were used per test in order to increase the probed surface area. An original holding system was custom-made to avoid sliding of the beads and ensure an isotropic compression during the test. Compression force as a function of displacement was measured as raw data. The stress-strain curve was then calculated to estimate the Young's modulus of the cartilage, taking into account the slope of the dataset in the linear regime, divided by the surface area of the sample and the number of the beads for normalization purposes. The calculation of the Young's modulus was made as described below:

$$E = \sigma / \varepsilon,$$

where $\sigma$ is the compression stress (kPa), E is the Young's modulus (kPa) and $\varepsilon$ the strain (no unit) corresponding to the normalized elongation.

**Immunoblotting**

Cells were lysed 30 min on ice in ice-cold RIPA buffer (Life Technologies), supplemented with phosphatase and protease inhibitors (Complete anti-protease cocktail; Roche). Protein (10μg) was separated by SDS-PAGE (BioRad) and transferred to PVDF membranes (Amersham). Blots were probed with anti-phospho-β-catenin (Cell Signaling; 5651T), TCF-4 (Cell Signaling; 2569), Axin1 (Cell Signaling; 2087), β-actin HRP (Sigma-Aldrich) and GADPH (Cell Signaling; 2118) (1:1000) followed by the HRP-Rabbit or Mouse conjugated antibodies (1:5000).

**Flow cytometry**

100'000 cells were fixed with PFA 4% then stained for CD73-CFS, CD90-APC, and CD105-PerCP (Cell Sigma) during 1 hour at RT in FACs buffer (BSA-Azide-PBS). A minimum of 10'000 living cells was acquired with a Gallios flow cytometer and the analysis was done using the FlowJo software. Results represent the average of three independent experiments for CD73 and CD90. Two independent experiments were performed for CD105. For the gating strategy, living cells were first selected, then single cells were identified based on FSC-W and FSC-A to remove doublets. The positive staining was defined based on the negative control IgG-CFS, IgG-APC, and IgG-PerCP for CD73-CFS, CD90-APC, and CD105-PerCP, respectively.

**RNASeq**

As previously described, the SR100 - libraries TruSeqHT stranded - Illumina HiSeq 4000 was used and the sequencing quality control was performed with FastQC v.0.11.5 (Cosset, E. et al. 2016. Biomaterials 107, 74-87). The quality distribution along the reads was evaluated and validated for all samples. The UCSC human hg38 reference was used to map the reads with STAR aligner v.2.5.3a to the reference genome. The average mapping rate was 93.54%. The transcriptome metrics were evaluated with the Picard tools v.1.141 and the differential expression analysis was performed with the statistical analysis R/Bioconductor package edgeR v. 3.18.1 (Gentleman, R. C. et al. 2004. Genome Biol 5, R80, Huber, W. et al. 2015. Nat Methods 12, 115-121). Briefly, the counts were normalized according to the library size and filtered. The genes having a count above 1 count per million reads (cpm) in at least 3 samples were kept for the analysis. The raw gene number of the set was 26'485. The poorly or not expressed genes were filtered out. The final data set consisted of 13'884 genes. The differentially expressed genes tests were done with exact Test using a negative binomial distribution. The differentially expressed genes p-values are corrected for multiple testing error with a 5% FDR (false discovery rate). The correction used is Benjamini-Hochberg (BH). The differentially expressed genes tests were done with edgeR using a negative binomial distribution. The p-value of differentially expressed genes was corrected for multiple testing error using Benjamini-Hochberg (BH) false discovery rate (FDR).

Panther analysis was used for determining the enrichment for each family of genes (number of entities) (Mi, H. et al. 2017. Nucleic Acids Res 45, D183-D189).

**RNA extraction and RT-PCR**

Isolation of total RNA was performed by using the RNeasy kit from Qiagen according to the manufacturer's instructions and was measured using a spectrometer. 500ng of total RNA was used to synthesize cDNA using the PrimeScript RT Reagent Kit (Takara) according to manufacturer's protocol.

Real-time PCR was performed with PowerUp SYBR Green Master Mix (Applied Biosystems) using the Quant-Studio 12K Flex Real-Time PCR System (Thermo Fisher Scientific) at the Genomic platform core facilities (University of Geneva). First, efficacy tests were performed for all primers for validation prior utilization. The relative level of each sample was normalized, at least, to 2 housekeeping genes (*ALAS1*). RT-PCR reactions were carried out, at least, in technical and biological triplicates, and the average cycle threshold (CT) values were determined.

**Transplantation of human Cartibeads in SCID/NOD mice for safety study**

Fifty-six male SCID/NOD mice were used to test the safety of the present standardized Cartibeads. Forty-four mice received human Cartibeads by subcutaneous implantation. One control group was used, where 8 animals received aggregated A549 adenocarcinoma cells, named beads (the number of mice per group was summarized in the Table 3).

**Table 3. List of donors Cartibeads transplanted into SCID mice.** Cartibeads from different donors and cell passage (from 3 to 6) were injected subcutaneously in SCID/NOD mice (n=8 to 14 mice per group). The adenocarcinoma cell line A549 was used as positive control and produced 100% of tumors. After animal euthanasia at 6 months, none of the mice showed weight loss due to Cartibead transplantation, abnormality by palpation or any sign of distress. In addition, no abnormality or evidence of tumor presence was observed in lungs, heart, liver, kidneys and spleen.

| Microtissues from | Cell passage | Mice transplanted | Tumor (6 months) |
|---|---|---|---|
| Donor #1 (M, 29) | p6 | 10 | 0 |
| Donor #2 (M, 47) | p3 | 10 | 0 |
| Donor #4 (M, 38) | P6 | 14 | 0 |
| Donor #12 (F,74) | P4 | 10 | 0 |
| Donor #13 (F,60 ) | P3 | 10 | 0 |
| A549 (adenocarcinoma) | | 8 | 100% (6 weeks) |

All implanted human Cartibeads were cultured according to the standardized 3-step method and transplanted in the same manner. General anaesthesia was achieved with 4% isoflurane followed by 2% isoflurane under mask with 5% oxygen. Local disinfection of the skin after shaving the back with 70% alcohol. Skin incision of 0.5 cm caudally from the occipital pole was performed. The produced tissues (Cartibeads and adenocarcinoma beads) of 200,000 cells each were implanted subcutaneously with a sterile pipet (1 bead/animal). The skin was then closed with surgical glue (Histoacryl, B. Braun Surgical S.A.). In order to locate the site and orientation of the sample of skin, the inventors performed a 4 cardinal point tattoo with a sterile 26-gauge needle and green tattoo ink. Mice in the control groups were euthanized at 4 to 6 weeks while the mice in the Cartibeads group were followed up to 6 months. Skin and organs were harvested and examined by two study members at each stage. Samples were all conserved 1/3 in formol 4% and 2/3 dry at -20°C. Animal procedures were approved by the Swiss Federal Veterinary Office (GE/12/18)

**Comparative genomic hybridization array**

Genomic stability was also determined comparing normal human dermal fibroblasts treated with FBS 10 % or PRP 10% for 6 days using array Comparative Genomic Hybridization (CGH). DNA was extracted using the QIAGEN QIAamp DNA Mini Kit (Qiagen, Hilden) according to the manufacturer's protocol. Array CGH was performed using the Agilent SurePrint G3 Human CGH Microarray kit 4_44K (design ID 014950) with 43 Kb overall median probe spacing (Agilent Technologies). Practical resolution was approximately 200 kb. Patient DNA and DNA of a sex-matched control (1 µg of each) was labelled with Cy3-dUTP and Cy5-dUTP, respectively (Sure Tag labeling kit, Agilent Technologies). Labeled products were purified by Amicon Ultra 30 K filters (Millipore). Hybridization was performed according to the protocol provided by Agilent. Patient and control DNA were pooled and hybridized with 2 mg of Human Cot-I DNA at 65 °C with rotation for 24 h. Arrays were analyzed using an Agilent SureScan Microarray scanner and the Agilent Feature Extraction software (v11.5), and results were presented by Agilent Genomic Workbench (v.7.0).

**Transplantation of autologous Cartibeads in minipig for efficacy study**

Six adult female minipigs (37-48 kg) were used in the study. The animals were anaesthetized with Sevoflurane and were given a prophylactic antibiotic intravenously (Cefazolin: 2 g/kg) 30 minutes prior to the incision. They were operated under complete narcosis and were extubated 5 minutes after skin closure. Skin disinfection was repeated three times and aseptic draping was performed with dischargeable surgery drapes.

Step 1 surgery

A super-lateral para-patellar approach was chosen for the first surgery. Cartilage harvesting was performed at the superolateral border of the lateral trochlear facette to prevent potential conflict with later cartilage lesions during the second surgical step. The cartilage biopsies were put into a culture medium E solution for further processing. A stepwise closure was performed. No bandage was applied.

Step 2 surgery

Five to 6 weeks after cartilage harvesting, the minipigs underwent the second surgery through a medial para-patellar tendon approach (Bonadio, M. B. et al. 2017. J Exp Orthop 4, 11). By this approach two lesions on the medial and lateral femoral trochlea each were created in all minipigs except one due to extent of the donor site. The size of the lesions were of a 6 mm in diameter and were created by a small curette, then filled completely by a single layer of 4-5 Cartibeads. For faster attachment, a thin layer of Tisseel (Baxter) was added to each defect. A fifth lesion was then created on the medial femoral condyle and filled with Cartibeads in identical manner. In each animal 1 lesion was kept empty at a different location as negative control lesion. A stepwise closure was performed. No bandage was applied.

Rehabilitation

Full weight-bearing was allowed as soon as the narcosis was finished with no restriction of any activity.

Euthanasia

Three minipigs were euthanized 3 months after the second surgery, and the other three were euthanized at 6 months. The operated knee was amputated, and the distal femur was put in formyl aldehyde solution for further analysis.
Animal procedures were approved by the Swiss Federal Veterinary Office (GE/60/18)

**RESULTS**

**Engineering and characterization of Cartibeads**

In this study, the inventors generated cartilage microtissues, named Cartibeads, with improved hyaline cartilage characteristics, which is defined by GAG content and collagen II expression. Cartibeads method is an innovative 3-step protocol (**Figure 1A**), consisting of (step 1) chondrocyte expansion in 2-dimensional culture (2D) characterized by cell dedifferentiation followed by (step 2) cell redifferentiation in 2D, defined as chondrogenic commitment, and finally by (step 3) a 3-dimensional culture (3D) allowing for Cartibeads formation by chondrocyte aggregation. Thus, human chondrocytes were enzymatically extracted from surgical waste material and cultured in 2D in atmospheric oxygen conditions (21%) in step 1 and 2 followed by 3D culture in low oxygen level (5%).
Chondrocytes become "fibroblastic" with stem cell characteristics in gene expression and express mesenchymal stem cell surface markers (CD105, CD90, CD73) (Figure 6). In three weeks, it is possible to obtain from 100 000 cells extracted, 60 to 100 million of cells after culture in the medium 1 (medium E).
The second step corresponds to the "reversion" of fibroblastic phenotype toward chondrocyte phenotype after massive cell amplification step. Cells are plated in redifferentiation (medium 2) for 3 to 7 days. At day 0, 2 million of cells are plated in a T75 cm2 flask to reach confluence and cells are kept confluent until day 4 to day 8 before detaching them for 3D culture. From a T75 cm2 flask, 6 to 10 million of cells are obtained in step 2. In comparison, in the first step in dedifferentiation medium, 12 to 16 million of cells are obtained in a T75 when they reach confluency. The removal of FGF-2 growth factor in redifferentiation medium (Medium R) decreases the growth of cells and allows the phenotype reversion and redifferentiation towards chondrocytes complete differentiation. Cells change their morphology in the second step of culture and become less elongated, larger, with apparent granular endoplasmic reticulum in the cytoplasm under optical microscopy, signaling thus a high level of protein synthesis activity. Chondrocytes start to re-express aggrecan, a proteoglycan associated with GAG (data RNAseq). At this second step in 2D culture, collagen II is not re-expressed yet.
In a third step, cells are detached from culture in the medium 2 (medium R) and seeded in 3D culture. 100 000 to 200 000 cells are dispensed in polypropylene conical 96 well plates in re-differentiation culture medium (medium 3, also named medium I). 96 well plates are centrifuged 5 min, 300 g to aggregate cells and form a pellet. 96 well plates are made in polypropylene material to avoid cells to stick at the surface of the well. Culture is incubated in hypoxia atmosphere (5% O2) to increase hyaline cartilage production during 15 days before using microtissues.
Cartibeads engineered from chondrocytes hold potentials as an ATMP for treating chondral lesions. Therefore, to evaluate its safety and efficacy the inventors used animal models (SCID mice and minipig) (**Figure 1B**). To better characterize the Cartibeads, histological qualitative analyses were undertaken. The presence of hyaline features with a homogeneous distribution of Safranin-O-stained GAG was shown and correlated with a strong immunodetection of type II collagen whereas type I collagen was faint **(Figure 1C).**
The inventors were able to engineer cartilage tissues (named cartibeads) of similar quality from donors up to 80 years of age, including from patients with osteoarthritis (OA). Quantitative analysis of Cartibeads revealed high GAG content, which was independent of patient age and of the joint's osteoarthritic status (**Figure 1D**, **Table 2**). The inventors measured an average of 40 μg of GAG/cartibead and average of GAG/DNA 50 (10;140) (**Figure 2**).

To characterize the biomechanical properties of Cartibeads, the inventors used an apparatus with an adapted custom-made mold to quantify the elasticity of Cartibeads following a compression test. The Young modulus was calculated by dividing the compression stress by the strain in the elastic part of the curve (normalized elongation) (Lee, J. K. et al. 2017, Nature Materials, 16:864-873). This method is commonly used for testing cartilage elasticity and reflects its extracellular matrix (ECM) composition. The inventors observed an increase of the resistance to the constraint with Cartibeads containing more GAG, suggesting a benefit of increasing GAG quantity (**Figure 1E**), which is in line with other published studies (Omelyanenko, N. P. et al. 2018, Cartilage, 1947603518798890).

Since Hypoxia-Inducible Factor 1-alpha (HIF-1α) is known to induce ECM constituents (Madeira et al. 2015, Trends in Biotechnology, 33:35-42), the inventors evaluated the effect of low oxygen levels during the 3D culture (5% oxygen) on the quality of Cartibeads compared to atmospheric conditions. As expected, the results showed an improvement of the hyaline quality of the cartilage beads in low oxygen conditions (**Figure 3A-C**). Of note, standard culture conditions at atmospheric conditions corresponds in fact to hyperoxia as the level of oxygen recorded in native cartilage tissue is between 0.5-5%, depending on depth (Lafont, J. E. 2010, Int. J. exp. Pathol. 91(2):99-106).

In cell therapy, the stability of cellular components of the material to be transplanted is essential. Thus, the inventors evaluated the stability of Cartibeads in different conditions as well as the proportion of viable cells. The inventors compared the amount of GAG in Cartibeads at day 0 after the end of maturation step, with Cartibeads kept at 4°C and 23°C for an additional 6 days (**Figure 4A**). The results showed that the concentration of GAG remained unchanged, 55 μg/cartibead on average, over time and indifferent temperature variation, indicating stability. Only a slight number of dead cells was detected (13%) in these conditions, suggesting a good survival of the chondrocytes in the Cartibeads (**Figure 4B**).

**Transcriptomic analyses of the 3-step Cartibead method identified low level expression of WNT genes as a key pathway involved quality of hyaline matrix.**

In an attempt to identify molecular pathways involved in increased production of hyaline cartilage, the 3-step Cartibead method was compared to a 2-step method, classically used in tissue engineering (**Figure 5A**). In terms of morphology, the 3-step method resulted in larger white-colored Cartibeads (diameter of 1-2 mm) than the 2-step method (diameter 0.5-1 mm) and a higher quantity of GAG/bead (**Figure 2A-C**). The improvement of Cartibeads hyaline quality in the 3-step method was mainly due to the introduction of an additional step after extensive cell expansion with FGF-2. This step, corresponding to a starvation step, used a medium "R", which does not contain FGF-2, and thereby facilitate the redifferentiation of chondrocytes and hyaline matrix production. In both methods, the final step consists in 3D cultures for 2 weeks in maturation medium I containing TGF-β3 supplementation (**Figure 5A**).

To understand the molecular mechanisms allowing Cartibead production, the inventors performed RNA sequencing (RNAseq) analysis using 3 donor samples at each critical step of both methods. RNA was extracted at different time points for the 3-step Cartibead method: (i) end of step 1 (amplified chondrocytes, medium E), (ii) end of step 2 in medium R, and (iii) after 15 days for step 3 (beads, medium I). For the 2-step method, 2-time points were selected: step 1 (amplified chondrocytes, medium E) and end of step 2 (beads, medium I). The inventors first compared cartilage microtissues generated by both methods in medium I (**Figure 5B** and **C**).

They observed significantly higher levels of genes involved in collagen and ECM degradation / formation along with ECM organization in Cartibeads (*COL1A1, COL2A1, COL4A1, MMP1, MMP13, MNIP11*). Differential gene expression analysis showed that the 3-step method induced much higher levels of type II collagen (*COL2A1*) and aggrecan (*ACAN*) in Cartibeads while type I collagen (*COL1A1*), specific of fibrocartilage was higher in the two-step method (**Figure 5C-F**). Increased levels of GAG confirmed the improved cartilage differentiation in the 3-step Cartibead method. (**Figure 5G**; Figure 2A-C). In the 3-step method (M1+M2+M3) *SOX9* (transcription factor involved in matrix production) is also increased (**Figure 7C**).

These data confirmed the dedifferentiation of chondrocytes in medium E (containing FGF-2) with the detection of *COL1A1* expression and the absence of hyaline cartilage markers such as type *COL2A1,* and *ACAN*, a GAG-associated protein (**Figure 5D-F**). The inventors confirmed the enrichment of dedifferentiated cells in medium E by flow cytometry, indicating that the proportion of cells expressing mesenchymal stem cell markers CD73, CD90 and CD 105, as defined proposed by the International Society for Cell Therapy (ISCT), at the end of expansion phase was more than 90% of the total cell population (**Figure 6A; Table 4**).

**Table 4: Flow cytometry analysis of dedifferentiated chondrocytes.** Table represents the flow cytometry analysis of dedifferentiated chondrocytes for CD73, CD90 and CD 105 from 3 donors.

|  | Donor 27 | Donor 14 | Donor 18 |
|---|---|---|---|
| CD73 | 99.7 | 99.8 | 99.6 |

(continued)

|  | Donor 27 | Donor 14 | Donor 18 |
|---|---|---|---|
| CD90 | 93.2 | 99.7 | 99.7 |
| CD105 | 89.2 | 92.9 | 90.7 |

Furthermore, multipotency characteristics of this population of dedifferentiated chondrocytes was confirmed by the fact that it has the capacity to differentiate towards other mesenchymal stem cell (MSC) derivative cells (osteocytes, adipocytes and chondrocytes) even if this potential remained lower compared to MSC (**Figure 6B**).

When the inventors compared the gene expression of cells cultured in medium R (redifferentiation step) to medium E (expansion step), they found higher levels of expression of genes involved in inflammation processes (interleukin and cytokine signaling along with interferon signaling) during redifferentiation. In parallel, the inventors found lower levels of expression of genes implicated in the cell cycle (*MI67, CDK, CCNB1*), indicating that hyaline matrix production requires a dynamic balance between cellular inflammation and tissue remodeling associated with an exit of the cell cycle (**Figure 7A**).

By comparing the expansion phase with the redifferentiation phase, the inventors observed high expression levels of *WNT5A, WNT5B* and *WNT7B* genes during the expansion phase (step 1, medium E, both methods) (**Figure 7B, D-F**). These genes were strongly downregulated during the redifferentiation step (medium R, 3-step method) and during 3D culture (medium I, both methods) except for *WNT5B*, which showed intermediate expression in 3D culture (step 3, medium I) (**Figure 7E**).

The expression of KI67 (a proliferative marker) and TCF4 (involved downstream the wnt/beta catenin pathway) is also decreased during the redifferentiation step **(Figure 7C).** Most importantly, after the dedifferentiation and redifferentiation phases (M1+M2), KI67 and WNT7B/WNT5B are diminished before sphere formation in maturation phase (M3). When cells are used to form spheroid directly from dedifferentiation phase (M1), they still highly expressed KI67 and WNT7B /WNT5B and results in less hyaline matrix production.

These results are in line with previous studies reporting an upregulation of WNT upon FGF-2 treatment (Buchtova, M. et al. 2015. Biochim Biophys Acta 1852, 839-850; Deng, Y. et al. 2019. Biomaterials 192, 569-578). Indeed, WNT signaling is involved in the stem-like phenotype (Buchtova, M. et al. 2015. Biochim Biophys Acta 1852, 839-850). Accordingly, FGF-2 removal in medium R led to WNT signaling downregulation during the redifferentiation phase. The inventors detected lower levels of *COL2A1* and *ACAN* expression in the 2-step method compared to the 3-step method (**Figure 5C-E**). However, they observed a comparable decrease of *WNT5A, WNT5B* and *WNT7B* genes in the 2-step method during the 3D differentiation phase in medium I (**Figure 7B**).

In order to validate the role of WNT pathway downregulation during the redifferentiation phase (medium R) (**Figure 1A**), the inventors replaced medium R by medium E supplemented with 10 $\mu$M of XAV-939, a WNT pathway inhibitor for 4 days (**Figure 7G**). Upon XAV-939 treatment, the inventors observed an increase of phospho-$\beta$-catenin and axin (**Figure 7G**), known indicators of WNT signaling blockade (Huang, S. M. et al. 2009. Nature 461, 614-620). Accordingly, similarly to medium R, pharmacological inhibition of WNT induced an increase of the *ACAN* and *COL2A1* expression in the 2-step method after 2D and 3D culture, (**Figure 7H, I**). As a result, pharmacological inhibition of WNT resulted in the presence of hyaline features of beads obtained in the 2-step method in presence of XAV-939, as confirmed by GAG Safranin-O staining (**Figure 7J**).

Altogether, transcriptomics analysis identified the involvement of WNT signaling pathway and its modulation in chondrocyte dedifferentiation and redifferentiation, together with *WNT5A, WNT5B,* and *WNT7B* acting as potential main mediators of this response (**Figure 7D-F**). Medium E supplemented with XAV-939 mimics the effect of medium R (**Figure 7K**), but the 3-step method naturally induced the downregulation of WNT genes in 7 days without the use of a costly pharmacological molecule.

***Ex vivo* feasibility study in a human cartilage defect**

In the context of a clinical application of Cartibeads, the inventors evaluated the capacity of Cartibeads to fuse together and integrate into a lesion generated in an *ex vivo* human knee joint. A lesion was created with an 8mm surgical punch biopsy and filled with 20 to 50 Cartibeads in dry condition. Cartibeads were kept 20 minutes in that condition, to facilitate the adhesion of Cartibeads to the lesion site and among each other (**Figure 8A, left and middle panel**), followed by the addition of medium I. Then, the whole specimen with its lesion filled with Cartibeads was cultivated for 1 month in rotation to facilitate high mass transfer of nutrients. One-month post implantation, the inventors analyzed the bone-cartilage tissue (**Figure 8A, right panel**) and performed Safranin-O staining at the level of the filled lesion (**Figure 8B**). The inventors observed maintenance of hyaline characteristic within the lesion and some level of integration of the Cartibeads with the native surrounding tissue and fusion of the Cartibeads among each other (**Figure 8B**).

**Preclinical safety studies of Cartibeads** *in vitro* **and** *in vivo*

Transplantation of cells expanded *in vitro* raises a problem of potential uncontrolled proliferation. Cartibeads safety was evaluated through preclinical studies. The CGH (comparative genomic hybridization) array analysis showed genetic stability of chondrocytes during cell amplification up to passage 11 (**Table 5**).

**Table 5: List of donors Cartibeads analyzed by CGH-array.** Genetic stability of chondrocytes was assessed after extensive expansion in medium E with the presence of FGF-2, by performing a comparative genomic hybridization (CGH) array analysis on ten donor samples cultured from passage 3 to 11.

| Donor analysed by CGH-array | Cell passage |
| --- | --- |
| Donor 3 (M, 29) | P4 & P8 |
| Donor 2 (M, 47) | P3 & P11 |
| Donor 20 (M, 38) | P4 |
| Donor 18 (M, 58) | P4 |
| Donor 23 (M, 63) | P3 (Y loss) |
| Donor 13 (F, 64) | P5 |
| Donor 8 (F, 65) | p4 |
| Donor 21 (M, 68) | P5 (Y loss) |
| Donor 14 (M, 69) | P5 |
| Donor 9 (M, 80) | P4 (Y loss) |

Consistent with previous studies, loss of chromosome Y is a common acquired mutation in normal aging of men and was observed in a fraction (43%) of older donors (Thompson, D. J. et al. 2019. Nature 575, 652-657; Stumm, M. et al. 2012. Osteoarthritis and cartilage 20, 1039-1045).
In the *in vivo* study, the inventors evaluated a potential tumorigenic effect of transplanted human Cartibeads in SCID mice. The Cartibeads did not expand and were mostly undetectable 6 months after implantation in SCID mice, thereby confirming absence of potential tumorigenicity over 6-months follow-up (**Figure 9A-C**, **Table 5**)**.**

**Preclinical efficacy study** *in vivo*

In order to move towards clinical application, the inventors performed a preclinical efficacy study in adult Göttingen minipigs, chosen for their cartilage maturity and for knee anatomy resembling that of human (Christensen, B. B. et al. 2015. Journal of experimental orthopaedics 2, 13; Pfeifer, C. G. et al. 2017. Tissue engineering. Part C, Methods 23, 745-753). Six females were used in the study. A first surgery was performed to collect a cartilage biopsy (~30 mg) from the knee to produce autologous minipig Cartibeads. A second surgery was performed to induce 4 to 5 lesions per animal / right knee and to transplant autologous Cartibeads. The minipigs were followed between 3 and 6 months (**Figure 10A; Figure 11**). Before transplantation, the amount of GAG in the minipig Cartibeads was quantified and ranged between 40 to 50 $\mu$g/bead on average (**Figure 10B**). At 3-month post-transplantation, the inventors confirmed the hyaline features of the lesions grafted with minipig Cartibeads by Safranin-O staining (**Figure 10C**). Similar results were obtained from the grafted lesions at 6-month post transplantation (**Figure 10D**). A complete fusion of Cartibeads together and their integration within the surrounding native cartilage and subchondral bone was observed in all minipigs at 3- and 6-months follow-up periods (**Figure 10C-D**). Collectively, the autologous Cartibeads proved their efficacy in terms of engraftment in the lesions, while preserving their hyaline quality. They also proved to be safe as neither a degeneration of the joint (**Figure 11, middle panel**), nor an ectopic or hypertrophic tissue formation were noted at macro inspection by two independent operators (data not shown).

**Discussion and conclusions**

The main achievement of the present study is a method that can reverse the loss of chondrocyte phenotype (dedifferentiation of chondrocytes) during expansion. This solves a key problem encountered in cell therapy using chondrocytes as the starting material. The present data demonstrates that a novel 3-step method can produce a high-quality cartilage with hyaline characteristics, regardless of the patient age and the joint's arthritic status.

The Cartibead method allows cell expansion from a very small sample of cartilage harvest (~30 mg in our preclinical minipig study), as opposed to 260 mg on average in conventional human chondrocyte-based cell therapy (Brittberg, M. 2008. Injury 39 Suppl 1, S40-49), which reduces donor site morbidity. The present method showed a ratio of GAG/Cartibead that is 20 times more than previously reported cartilage microtissues, suggesting that these cartilage microtissues are less hyaline and contained more fibrocartilage (Bartz, C. et al. 2016. J Transl Med 14, 317). Consistent with those results, the inventors obtained on average a ratio of GAG/DNA at least three times higher than other published method (**Figure 2B**) (Mumme, M. et al. 2016. Lancet 388, 1985-1994; Dang, P. N., Solorio, L. D. & Alsberg, E. 2014. Tissue engineering. Part A 20, 3163-3175).

Redifferentiation was due to removal of FGF-2. However, its removal in 3D culture was not sufficient to induce hyaline matrix synthesis in the 2-step method. Redifferentiation of chondrocytes most likely requires cell adhesion to a matrix coated-flask and induction of specific cell signaling pathways in 2D culture. Starvation of FGF-2 shuts down genes involved in multipotency and sternness, including several genes of the WNT signaling pathway. WNT signaling is involved in both inhibition and stimulation of chondrogenic differentiation of adult progenitor cells (Day, T. F. et al. 2005. Developmental cell 8, 739-750; Hill, T. P., et al. 2005. Developmental cell 8, 727-738; Hu, H. et al. 2005. Development, 132, 49-60). As shown in embryos, high levels of WNT/β-catenin signaling inhibit chondrogenic differentiation of stem cells, while down-regulation of this pathway induces chondrogenesis (Hartmann, C. 2007. Molecules and cells 24, 177-184; Johnson, M. L. & Rajamannan, N. 2006. Reviews in endocrine & metabolic disorders 7, 41-49; Westendorf, J. J., Kahler, R. A. & Schroeder, T. M. 2004. Gene 341, 19-39).

In the present study, the inventors identified *WNT5A, WNT5B* and *WNT7B* as probable WNT isoforms involved in this mechanism. In parallel with the downregulation of the WNT pathway, the inventors observed an increase in expression of genes involved in the inflammatory pathway (interleukins, cytokines). In wound healing and tissue repair, a dynamic balance between pro- and anti-inflammatory factors is essential for effective healing (Gerhard T. Laschober et al. 2011. Rejuvenation Research 14, 119-131; Bosurgi, L. et al. 2017. Science 356, 1072-1076). Accordingly, the present data suggest that inflammation is a prerequisite for tissue regeneration associated with hyaline matrix biosynthesis (Karin, M. & Clevers, H.2016. Nature 529, 307-315).

Human Cartibeads did not proliferate when implanted in SCID mice and even disappeared after 6 months, which is consistent with similar studies (Zscharnack, M. et al. 2015. J Transl Med 13, 160). Moreover, autologous transplantation of Cartibeads in minipigs showed stable integration into lesions, maintained high levels of GAG and successfully repaired cartilage damage at 6 months after transplantation. In summary, the inventors showed the feasibility of Cartibead transplant containing high level of GAG in a large animal model, supporting its potentials for a long-term cartilage repair. Consequently, the Cartibeads represent a breakthrough in the field of cartilage repair and can now enter a clinical trial phase I (First-in-Human) for autologous cartilage transplantations in patients with cartilage damages.

**Claims**

1. A method for *in vitro* production of hyaline cartilage tissue comprising:

   i) culturing chondrocytes on an adherent culture system in a dedifferentiation culture medium that activates Wnt signaling pathway to obtain chondrocytes with a morphology of fibroblastic-like cells;
   ii) culturing said fibroblastic-like chondrocytes on an adherent culture system in a redifferentiation culture medium that inactivates Wnt signaling pathway to obtain chondrocytes with full capacity to resynthesize hyaline matrix;
   iii) culturing said chondrocytes obtained in step ii) in a three-dimensional culture system in an induction/maturation culture medium that maintains the inactivation of Wnt signaling pathway.

2. The method of claim **1**, wherein said dedifferentiation culture medium comprises FGF-2 and said redifferentiation culture medium and induction/maturation culture medium are FGF-2 free media.

3. The method of claim **1** or **2**, wherein said dedifferentiation culture medium comprises at least one growth factor selected from the group consisting of: FGF-2, PDGF-BB, TGF-β and EGF, preferably said dedifferentiation culture medium comprises FGF-2.

4. The method according to any one of claims **1** to **3,** wherein said redifferentiation culture medium and said induction/maturation culture medium comprise TGF-β, preferably TGF-β3, more preferably TGF-β3 and FGF-7.

5. The method according to any one of claims **1** to **4**, wherein said redifferentiation culture medium comprises platelet lysate.

6. The method according to any one of claims **1** to **5**, wherein said dedifferentiation culture medium, redifferentiation culture medium and/or induction/maturation culture medium comprise(s) serum.

7. The method according to any one of claims **1** to **6**, wherein said induction/maturation culture medium comprises at least one component selected from the group consisting of: insulin, IGF-1, BMP-2, selenium, transferrin, and ethanolamine.

8. The method according to any one of claims **1** to **7**, wherein said chondrocytes are cultured in step iii) in hypoxia atmosphere comprising less than 10% $O_2$ (v/v).

9. The method according to any one of claims **1** to **8**, wherein said chondrocytes are cultured in step i) during 10 to 15 days, in step ii) during 4 to 8 days, and/or in step iii) during 10 to 15 days.

10. The method according to any one of claims **1** to **9**, wherein said chondrocytes of step i) are chondrocytes isolated from a subject, preferably from cartilage tissue of a human or horse subject.

11. An engineered cartilage tissue in a form of spheroid presenting a glycosaminoglycan (GAG) content per spheroid between 10 and 100 $\mu$g/spheroid obtainable by the method of any of claims **1** to **10.**

12. A pharmaceutical composition comprising the engineered cartilage tissue of claim **11** and one or more pharmaceutically acceptable excipients.

13. The engineered cartilage tissue of claim **11** or the pharmaceutical composition of claim **12** for use in the treatment of cartilage defects and cartilage degenerative disease in a subject in need thereof.

14. The engineered cartilage tissue or pharmaceutical composition for use according to claim **13,** wherein said engineered cartilage tissue or pharmaceutical composition is for use in autologous transplantation in a subject in need thereof.

15. The engineered cartilage tissue or pharmaceutical composition for use according to claim **13**, wherein said engineered cartilage tissue or pharmaceutical composition is for use in allogenic transplantation in a subject in need thereof.

16. A method of screening molecules inhibiting the cartilage degenerative process comprising:

i) contacting the engineered cartilage tissue of claim **11** with one or more candidate molecules, and
ii) selecting molecules inhibiting the cartilage degenerative process.

**Patentansprüche**

1. Verfahren zur Herstellung von hyalinem Knorpelgewebe *in vitro,* umfassend:

i) Kultivieren von Chondrozyten auf einem adhärenten Kultursystem in einem Dedifferenzierungskulturmedium, das den Wnt-Signalweg aktiviert, um Chondrozyten mit einer Morphologie von fibroblastenähnlichen Zellen zu erhalten;
ii) Kultivieren der fibroblastenähnlichen Chondrozyten auf einem adhärenten Kultursystem in einem Redifferenzierungskulturmedium, das den Wnt-Signalweg inaktiviert, um Chondrozyten mit der vollen Fähigkeit zur Resynthese von hyaliner Matrix zu erhalten;
iii) Kultivieren der in Schritt ii) erhaltenen Chondrozyten in einem dreidimensionalen Kultursystem in einem Induktions-/Reifungskulturmedium, das die Inaktivierung des Wnt-Signalwegs aufrechterhält.

2. Verfahren nach Anspruch **1**, wobei das Dedifferenzierungskulturmedium FGF-2 umfasst und das Redifferenzierungskulturmedium und das Induktions-/Reifungskulturmedium FGF-2-freie Medien sind.

3. Verfahren nach Anspruch **1** oder **2**, wobei das Dedifferenzierungskulturmedium mindestens einen Wachstumsfaktor umfasst, der aus der Gruppe ausgewählt ist, die aus Folgendem besteht: FGF-2, PDGF-BB, TGF-β und EGF, vorzugsweise umfasst das Dedifferenzierungskulturmedium FGF-2.

4. Verfahren nach einem der Ansprüche **1** bis **3**, wobei das Redifferenzierungskulturmedium und das Induktions-/Reifungskulturmedium TGF-β, vorzugsweise TGF-β3, noch bevorzugter TGF-β3 und FGF-7 umfassen.

5. Verfahren nach einem der Ansprüche **1** bis **4**, wobei das Redifferenzierungskulturmedium Thrombozytenlysat umfasst.

6. Verfahren nach einem der Ansprüche **1** bis **5**, wobei das Dedifferenzierungskulturmedium, das Redifferenzierungskulturmedium und/oder das Induktions-/Reifungskulturmedium Serum umfasst/umfassen.

7. Verfahren nach einem der Ansprüche **1** bis **6**, wobei das Induktions-/Reifungskulturmedium mindestens eine Komponente umfasst, die aus der Gruppe ausgewählt ist, die aus Folgendem besteht: Insulin, IGF-1, BMP-2, Selen, Transferrin und Ethanolamin.

8. Verfahren nach einem der Ansprüche **1** bis **7,** wobei die Chondrozyten in Schritt iii) in einer Hypoxie-Atmosphäre kultiviert werden, die weniger als 10 % $O_2$ (v/v) umfasst.

9. Verfahren nach einem der Ansprüche **1** bis **8,** wobei die Chondrozyten in Schritt i) während 10 bis 15 Tagen, in Schritt ii) während 4 bis 8 Tagen und/oder in Schritt iii) während 10 bis 15 Tagen kultiviert werden.

10. Verfahren nach einem der Ansprüche **1** bis **9**, wobei es sich bei den Chondrozyten von Schritt i) um Chondrozyten handelt, die aus einem Subjekt, vorzugsweise aus Knorpelgewebe eines Menschen oder Pferdes, isoliert wurden.

11. Konstruiertes Knorpelgewebe in Form eines Sphäroids, das einen Gehalt an Glycosaminoglycan (GAG) pro Sphäroid zwischen 10 und 100 μg/Sphäroid aufweist, erhältlich durch das Verfahren nach einem der Ansprüche **1** bis **10.**

12. Pharmazeutische Zusammensetzung, umfassend das konstruierte Knorpelgewebe nach Anspruch **11** und einen oder mehrere pharmazeutisch annehmbare Hilfsstoffe.

13. Konstruiertes Knorpelgewebe nach Anspruch **11** oder die pharmazeutische Zusammensetzung nach Anspruch **12** zur Verwendung bei der Behandlung von Knorpeldefekten und degenerativen Knorpelerkrankungen bei einem Subjekt, das dies benötigt.

14. Konstruiertes Knorpelgewebe oder pharmazeutische Zusammensetzung zur Verwendung nach Anspruch **13,** wobei das konstruierte Knorpelgewebe oder die pharmazeutische Zusammensetzung zur Verwendung bei der autologen Transplantation bei einem Subjekt, das dies benötigt, bestimmt ist.

15. Konstruiertes Knorpelgewebe oder pharmazeutische Zusammensetzung zur Verwendung nach Anspruch **13,** wobei das konstruierte Knorpelgewebe oder die pharmazeutische Zusammensetzung zur Verwendung bei der allogenen Transplantation bei einem Subjekt, das dies benötigt, bestimmt ist.

16. Verfahren zum Screening von Molekülen, die den Knorpeldegenerationsprozess hemmen, umfassend:

i) Inkontaktbringen des konstruierten Knorpelgewebes nach Anspruch **11** mit einem oder mehreren Kandidatenmolekülen, und
ii) Auswählen von Molekülen, die den degenerativen Prozess des Knorpels hemmen.

**Revendications**

1. Une méthode de production *in vitro* de tissu cartilagineux hyalin comprenant :

i) la culture de chondrocytes sur un système de culture adhérent dans un milieu de culture de dédifférenciation qui active la voie de signalisation Wnt pour obtenir des chondrocytes avec une morphologie de cellules de type fibroblastique ;
ii) la culture desdits chondrocytes de type fibroblastique sur un système de culture adhérent dans un milieu de

culture de rediﬀérenciation qui inactive la voie de signalisation Wnt pour obtenir des chondrocytes avec la pleine capacité à resynthétiser la matrice hyaline ;

iii) la culture desdits chondrocytes obtenus à l'étape ii) dans un système de culture tridimensionnel dans un milieu de culture d'induction/maturation qui maintient l'inactivation de la voie de signalisation Wnt.

2. La méthode selon la revendication **1,** dans laquelle ledit milieu de culture de dédifférenciation comprend du FGF-2 et lesdits milieu de culture de rediﬀérenciation et milieu de culture d'induction/maturation sont des milieux exempts de FGF-2.

3. La méthode selon la revendication **1** ou **2,** dans laquelle ledit milieu de culture de dédifférenciation comprend au moins un facteur de croissance sélectionné parmi le groupe consistant en : FGF-2, PDGF-BB, TGF-β et EGF, de préférence ledit milieu de culture de dédifférenciation comprend du FGF-2.

4. La méthode selon l'une quelconque des revendications 1 à 3, dans laquelle ledit milieu de culture de rediﬀérenciation et ledit milieu de culture d'induction/maturation comprennent du TGF-β, de préférence du TGF-β3, et plus préférentiellement du TGF-β3 et du FGF-7.

5. La méthode selon l'une quelconque des revendications **1** à **4,** dans laquelle ledit milieu de culture de rediﬀérenciation comprend du lysat plaquettaire.

6. La méthode selon l'une quelconque des revendications **1** à **5,** dans laquelle lesdits milieu de culture de dédifférenciation, milieu de culture de rediﬀérenciation et/ou milieu de culture d'induction/maturation comprennent du sérum.

7. La méthode selon l'une quelconque des revendications **1** à **6,** dans laquelle ledit milieu de culture d'induction/maturation comprend au moins un composant sélectionné parmi le groupe consistant en : insuline, IGF-1, BMP-2, sélénium, transferrine et éthanolamine.

8. La méthode selon l'une quelconque des revendications **1** à **7,** dans laquelle lesdits chondrocytes sont cultivés à l'étape iii) dans une atmosphère hypoxique comprenant moins de 10 % d'$O_2$ (v/v).

9. La méthode selon l'une quelconque des revendications **1** à **8,** dans laquelle lesdits chondrocytes sont cultivés à l'étape i) durant 10 à 15 jours, à l'étape ii) durant 4 à 8 jours, et/ou à l'étape iii) durant 10 à 15 jours.

10. La méthode selon l'une quelconque des revendications **1** à **9,** dans laquelle lesdits chondrocytes de l'étape i) sont des chondrocytes isolés d'un sujet, de préférence du tissu cartilagineux d'un sujet humain ou équin.

11. Un tissu cartilagineux fabriqué sous forme de sphéroïde présentant une teneur en glycosaminoglycanes (GAG) par sphéroïde entre 10 et 100 μg/sphéroïde, obtenable par la méthode selon l'une quelconque des revendications **1** à **10.**

12. Une composition pharmaceutique comprenant le tissu cartilagineux fabriqué selon la revendication **11** et un ou plusieurs excipients pharmaceutiques acceptables.

13. Le tissu cartilagineux fabriqué selon la revendication **11** ou la composition pharmaceutique selon la revendication **12** pour son utilisation dans le traitement des défauts du cartilage et des maladies dégénératives du cartilage chez un sujet en ayant besoin.

14. Le tissu cartilagineux fabriqué ou composition pharmaceutique pour son utilisation selon la revendication **13,** dans laquelle ledit tissu cartilagineux fabriqué ou composition pharmaceutique est pour utilisation dans une transplantation autologue chez un sujet en ayant besoin.

15. Le tissu cartilagineux fabriqué ou composition pharmaceutique pour son utilisation selon la revendication **13,** dans laquelle ledit tissu cartilagineux fabriqué ou composition pharmaceutique est pour utilisation dans une transplantation allogénique chez un sujet en ayant besoin.

16. Une méthode de criblage de molécules inhibant le processus de dégénérescence du cartilage comprenant :

i) la mise en contact du tissu cartilagineux fabriqué selon la revendication **11** avec une ou plusieurs molécules candidates, et

ii) la sélection des molécules inhibant le processus de dégénérescence du cartilage.

**FIG. 1A-B**

FIG. 1C-E

**FIG. 2**

**FIG. 3**

**FIG. 4**

**a**

2-step method

**Medium E**
**(+) FGF-2**

**Medium I**
**(-) FGF-2, (+) TGF-beta 3**

day 1

day 16

**b**

**Cartibeads : 3- steps vs 2-steps**

Negative log FC

| | |
|---|---|
| Platelet degranulation | *** |
| TP53 regulates transcription of cell cycle genes | *** |
| IL-4 and IL-13 signaling | *** |

IL-4/-13 signaling

Positive Log FC

| | |
|---|---|
| RHO GTPases activate PAKs | ** |
| Ephrin signaling | *** |
| TP53 regulation of transcription of genes involved in... | *** |
| Elastic fibre formation | *** |
| ECM organization | *** |
| Collagen biosynthesis | *** |
| Collagen chain trimerization | *** |
| Collagen degradation, formation | *** |

Collagen biosynthesis

0    5    10    15    20    25    30    35

nb of entities

**FIG. 5A-B**

**FIG. 5C-G**

**FIG. 6**

FIG. 7A-B

**FIG. 7C**

**FIG. 7 D-K**

a

b

**FIG. 8**

a

Cartibead 2 months post-implantationt

Adenocarcinoma 6 weeks post-implantation

b

Tumor growth *in vivo*

c  Native cartilage control

Before implantation

Post implantation

**FIG. 9**

**FIG. 10**

**FIG. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6573099 B
- US 6506559 B
- WO 0136646 A
- WO 9932619 A
- WO 0168836 A

### Non-patent literature cited in the description

- **KON E et al.** *The American journal of sports medicine,* 2009, vol. 37 (1), 33-41
- **EKKERS JE et al.** *Osteoarthritis Research Society,* 2013, vol. 21 (7), 950-956
- **ARMOIRY X et al.** *Pharmacoeconomics,* 2019, vol. 37, 879-886
- **MUNIRAH, S. et al.** *Tissue & cell,* 2010, vol. 42, 282-292
- **WU, L. et al.** *Tissue Eng Part C Methods,* 2014, vol. 20, 160-168
- **BENYA, P. D et al.** *Cell,* 1978, vol. 15, 1313-1321
- **BENYA, P. D. ; SHAFFER, J. D.** *Cell,* 1982, vol. 30, 215-224
- *Tissue Engineering,* vol. 10 (5-6), 762-770
- **BRITTBERG, M.** *Injury,* 2018, vol. 39 (1), 40-49
- **BARTZ, C. et al.** *J Transl Med,* 2016, vol. 14, 317
- **MUMME, M. et al.** *Lancet,* 2016, vol. 388, 1985-1994
- **DANG, P. N et al.** *Tissue engineering.,* 2014, vol. 20, 3163-3175
- **NEGORO, T et al.** *npj Regenerative Medicine,* 2018, vol. 3 (17
- **MADEIRA, C et al.** *Trends in biotechnology,* 2015, vol. 33, 35-42
- *CHEMICAL ABSTRACTS,* 7447-41-8
- *CHEMICAL ABSTRACTS,* 252917-06-9
- *CHEMICAL ABSTRACTS,* 280744-09-4
- *CHEMICAL ABSTRACTS,* 667463-62-9
- *CHEMICAL ABSTRACTS,* 144678-63-7
- *CHEMICAL ABSTRACTS,* 3289-8625
- *CHEMICAL ABSTRACTS,* 294891-81-9
- *CHEMICAL ABSTRACTS,* 335206-54-7
- *CHEMICAL ABSTRACTS,* 50-65-7
- *CHEMICAL ABSTRACTS,* 38194-50-2
- *CHEMICAL ABSTRACTS,* 7187-62-4
- *CHEMICAL ABSTRACTS,* 34211-25-1
- *CHEMICAL ABSTRACTS,* 38965-74-1
- *CHEMICAL ABSTRACTS,* 3650-09-7
- *CHEMICAL ABSTRACTS,* 847591-62-2
- *CHEMICAL ABSTRACTS,* 113906-27-7
- *CHEMICAL ABSTRACTS,* 19881-70-0
- *CHEMICAL ABSTRACTS,* 1427782-89-5
- *CHEMICAL ABSTRACTS,* 1243243-89-1
- *CHEMICAL ABSTRACTS,* 1243244-14-5
- *CHEMICAL ABSTRACTS,* 1638250-96-0
- *CHEMICAL ABSTRACTS,* 284028-89-3
- *CHEMICAL ABSTRACTS,* 1140964-99-3
- **COSSET, E. et al.** *Biomaterials,* 2016, vol. 107, 74-87
- **GENTLEMAN, R. C. et al.** *Genome Biol,* 2004, vol. 5, R80
- **HUBER, W. et al.** *Nat Methods,* 2015, vol. 12, 115-121
- **MI, H. et al.** *Nucleic Acids Res,* 2017, vol. 45, D183-D189
- **BONADIO, M. B. et al.** *J Exp Orthop,* 2017, vol. 4, 11
- **LEE, J. K. et al.** *Nature Materials,* 2017, vol. 16, 864-873
- **MADEIRA et al.** *Trends in Biotechnology,* 2015, vol. 33, 35-42
- **LAFONT, J. E.** *Int. J. exp. Pathol.,* 2010, vol. 91 (2), 99-106
- **BUCHTOVA, M. et al.** *Biochim Biophys Acta,* 2015, vol. 1852, 839-850
- **DENG, Y. et al.** *Biomaterials,* 2019, vol. 192, 569-578
- **HUANG, S. M. et al.** *Nature,* 2009, vol. 461, 614-620
- **THOMPSON, D. J. et al.** *Nature,* 2019, vol. 575, 652-657
- **STUMM, M. et al.** *Osteoarthritis and cartilage,* 2012, vol. 20, 1039-1045
- **CHRISTENSEN, B. B. et al.** *Journal of experimental orthopaedics,* 2015, vol. 2, 13
- **PFEIFER, C. G. et al.** *Tissue engineering,* 2017, vol. 23, 745-753
- **BRITTBERG, M.** *Injury,* 2008, vol. 39 (1), 40-49
- **DANG, P. N. ; SOLORIO, L. D. ; ALSBERG, E.** *Tissue engineering.,* 2014, vol. 20, 3163-3175

- **DAY, T. F. et al.** *Developmental cell,* 2005, vol. 8, 739-750
- **HILL, T. P. et al.** *Developmental cell,* 2005, vol. 8, 727-738
- **HU, H. et al.** *Development,* 2005, vol. 132, 49-60
- **HARTMANN, C.** *Molecules and cells,* 2007, vol. 24, 177-184
- **JOHNSON, M. L ; RAJAMANNAN, N.** *Reviews in endocrine & metabolic disorders,* 2006, vol. 7, 41-49
- **WESTENDORF, J. J. ; KAHLER, R. A. ; SCHROEDER, T. M.** *Gene,* 2004, vol. 341, 19-39
- **GERHARD T. LASCHOBER et al.** *Rejuvenation Research,* 2011, vol. 14, 119-131
- **BOSURGI, L. et al.** *Science,* 2017, vol. 356, 1072-1076
- **KARIN, M. ; CLEVERS, H.** *Nature,* 2016, vol. 529, 307-315
- **ZSCHARNACK, M. et al.** *J Transl Med,* 2015, vol. 13, 160